# EUROPEAN PATENT APPLICATION

(11) **EP 4 230 615 A1**
(43) Date of publication of application: **23.08.2023**
(21) Application number: 21880225.4
(22) Date of filing: 15.10.2021
(51) Int. Cl.: C07C 211/54, H01L 51/50

(54) **ARYLAMINE COMPOUND, ORGANIC ELECTROLUMINESCENT ELEMENT, AND ELECTRONIC DEVICE**

(30) Priority: 16.10.2020 JP 2020174335
(71) Applicant: Hodogaya Chemical Co., Ltd., Tokyo, 105-0021 (JP)
(72) Inventor: KASE, Kouki, Tokyo 104-0028 (JP); IZUMIDA, Junichi, Tokyo 104-0028 (JP); HAYASHI, Shuichi, Tokyo 104-0028 (JP)
(74) Representative: Forstmeyer, Dietmar
(86) International application number: PCT/JP2021/038297
(87) International publication number: WO 2022/080490

(57) **Abstract**

It is an object of the present invention to provide, as a material for a highly efficient and highly durable organic EL device, an organic compound with excellent properties including excellent hole-injecting/transporting performance, electron-blocking capability, and high stability in the form of a film. It is another object thereof to provide a highly efficient and highly durable organic EL device by using this compound. The present invention is directed to an arylamine compound represented by a general formula (1) below: 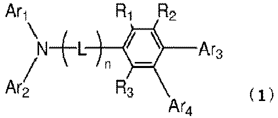 where Ar₁ and Ar₂ represent a substituted or unsubstituted aromatic hydrocarbon group or the like, Ar₃ and Ar₄ represent a substituted or unsubstituted phenyl group or the like, L represents a substituted or unsubstituted divalent aromatic hydrocarbon group or the like, Ri to R₃ represent a hydrogen atom, for example, and n is an integer of 1 to 2.

## Description

### Technical Field

The present invention relates to a compound suitable for organic electroluminescence devices (hereinafter referred to simply as "organic EL devices"), which are self-light emitting devices favorably used in various display device, and the organic EL device, and more particularly relates to an arylamine compound and an organic EL device including the compound.

### Background Art

Since organic EL devices are self-emissive devices, they have larger brightness and better viewability than devices including liquid crystals, and can provide a clearer display. For these reasons, active studies have been carried out on organic EL devices.

In 1987, C. W. Tang et al. of Eastman Kodak Company developed a device having a layered structure in which various functions were assigned to different materials, and thus made a practical organic EL device including organic materials. They made an organic EL device having a layered structure including a layer of a fluorescent substance capable of transporting electrons and a layer of an organic substance capable of transporting holes, and injected both charges into the layer of the fluorescent substance to thereby cause the layer to emit light, and the organic EL device thus achieved a luminance as high as 1,000 cd/m² or more at a voltage of 10 V or less (see Patent Literatures 1 and 2, for example).

Organic EL devices have been heretofore much improved to put them to practical use. Electroluminescent devices have been suggested in which an anode, a hole-injecting layer, a hole-transporting layer, a light-emitting layer, an electron-transporting layer, an electron-injecting layer, and a cathode are sequentially provided on a substrate to subdivide various functions in the multi-layered structure even further, and such electroluminescent devices successfully have high efficiency and durability (see Non-Patent Literature 1, for example).

To further increase luminous efficacy, attempts have been made to utilize triplet excitons, and the utilization of phosphorescent compounds has been investigated (see Non-Patent Literature 2, for example). Moreover, devices that utilize light emission by thermally activated delayed fluorescence (TADF) have also been developed. In 2011, Adachi et al. from Kyushu University achieved a result of an external quantum efficiency of 5.3% by a device including a thermally activated delayed fluorescence material (see Non-Patent Literature 3, for example).

The light-emitting layer can also be prepared by doping a charge-transporting compound, generally called a host material, with a fluorescent compound, a phosphorescent light emitting compound, or a material that radiates delayed fluorescence. As stated in the non-patent literature above, the selection of the organic materials in an organic EL device greatly affects the characteristics of that device, such as efficiency and durability (see Non-Patent Literature 2, for example).

In an organic EL device, the charges injected from both electrodes recombine in the light-emitting layer, thereby producing light emission, and how efficiently the both charges, i.e., the holes and the electrons, are transported to the light-emitting layer is of importance. Thus, the device needs to have excellent carrier balance. Accordingly, the probability of recombinations of holes and electrons in the light-emitting layer can be increased by using a material that improves hole-injecting capability, that is, the ability to supply holes injected from the anode to the light-emitting layer and improves electron-blocking capability, that is, the ability to block electrons injected from the cathode, and furthermore, higher luminous efficacy can be achieved by confining excitons generated in the light-emitting layer. For these purposes, the functions of the hole-transporting material are important, and there is a demand for a hole-transporting material having high hole-injecting capability, high hole mobility, high electron-blocking capability, and high durability against electrons.

Moreover, heat resistance and amorphousness of the materials are also important for lifespan of the device. A material with low heat resistance thermally decomposes, due to heat generated during the operation of the device, even at a low temperature, and thus the material deteriorates. In film made of a material with low amorphousness crystallization thereof is caused even in a short period of time to result in deterioration of the device. Thus, the materials to be used are required to have high heat resistance and good amorphousness.

N,N'-diphenyl-N,N'-di(α-naphthyl)benzidine (NPD) and various aromatic amine derivatives are known as hole-transporting materials that have been heretofore used for organic EL devices (see Patent Literatures 1 and 2, for example). NPD has good hole-transporting capability, but has a glass transition point (Tg), which is a measure of heat resistance, as low as 96°C. Such a glass transition point cause a deterioration of the device characteristics due to crystallization of NPD under high-temperature conditions (see Non-Patent Literature 4, for example).

Moreover, although the aromatic amine derivatives disclosed in Patent Literatures 1 and 2 include compounds having an excellent hole mobility of 10⁻³ cm²/Vs or higher, the electron-blocking capability thereof is insufficient. Thus, when using such a compound, some electrons pass through the light-emitting layer, and unfortunately, no increase in luminous efficacy can be expected. Accordingly, in order to further increase the efficacy, materials that have higher electron-blocking capability, higher stability in the form of a thin film, and higher heat resistance are needed. Furthermore, although aromatic amine derivatives with high durability have been reported (see Patent Literature 3, for example), these aromatic amine derivatives are used as charge-transporting materials for a photoconductor for electrophotography, and there are no precedents of application to an organic EL device.

With a view to addressing these issues, arylamine compounds having a substituted carbazole structure or a triarylbenzene structure have been suggested as compounds improved in properties such as heat resistance and hole-injecting capability (see Patent Literatures 4 and 5, for example). Although devices including such a compound in a hole-injecting layer or a hole-transporting layer have improved lifespan and luminous efficacy, the performance thereof are still insufficient. Therefore, there is demand for a further decrease in driving voltage, a further increase in luminous efficacy, and an increase in lifespan of devices.

### Citation List

### Patent Literatures

Patent Literature 1: US 5792557
Patent Literature 2: US 5639914
Patent Literature 3: US 7759030
Patent Literature 4: US 8021764
Patent Literature 5: US 10818844
Patent Literature 6: EP 2684932

### Non-Patent Literatures

Non-Patent Literature 1: Proceedings of the 9th Meeting of the Japan Society of Applied Physics, pp. 55-61 (2001)
Non-Patent Literature 2: Proceedings of the 9th Meeting of the Japan Society of Applied Physics, pp. 23-31 (2001)
Non-Patent Literature 3: Appl. Phys. Let., 98, 083302 (2011)
Non-Patent Literature 4: Proceedings of the 3rd Meeting of the Japan OLED Forum, pp. 13-14 (2006)

### Summary of Invention

An object of the present invention is to provide a material for a highly efficient and highly durable organic EL device, and specifically, a material for an organic EL device with (1) excellent hole-injecting/transporting performance, (2) electron-blocking capability, (3) high stability in the form of a thin film, and (4) excellent durability.

Another object of the present invention is to provide, by using the material as described above, an organic EL device with (1) high luminous efficacy and high power efficiency, (2) a low voltage for the start of light emission and a low actual driving voltage, and (3) a long lifespan.

To achieve the above-described object, the inventors of the present invention have focused on the fact that an arylamine compound having a triarylphenyl structure is excellent in terms of hole-injecting/transporting performance, electron-blocking capability, and stability and durability in the form of a thin film, and have found that the properties of the material can be significantly improved by introducing a substituent at a specific position to optimize the structure. They have also found that, by using this material, it is possible to realize an organic EL device with improved luminous efficacy and power efficiency, a low voltage for the start of light emission and a low actual driving voltage, and an increased lifespan longer than that of conventional organic EL devices. In this way, the present invention has been accomplished.
1) Specifically, the present invention is directed to an arylamine compound represented by a general formula (1) below. 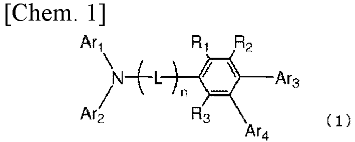
   where An and Ar₂ are the same or different, and each represent a substituted or unsubstituted aromatic hydrocarbon group, a substituted or unsubstituted aromatic heterocyclic group, or a substituted or unsubstituted fused polycyclic aromatic group;
   Ar₃ and Ar₄ are the same or different, and each represent a substituted or unsubstituted phenyl group, a substituted or unsubstituted biphenyl group, a substituted or unsubstituted naphthyl group, a substituted or unsubstituted triphenylsilyl group, a substituted or unsubstituted carbazolyl group, or a substituted or unsubstituted phenanthrenyl group,
   L represents a substituted or unsubstituted divalent aromatic hydrocarbon group, a substituted or unsubstituted divalent aromatic heterocyclic group, or a substituted or unsubstituted divalent fused polycyclic aromatic group;
   R₁ to R₃ are the same or different, and each represent a hydrogen atom, a deuterium atom, a fluorine atom, a chlorine atom, a cyano group, a nitro group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group; and
   n is an integer of 1 or 2, where, when n is 2, L is the same or different.
2) The present invention is further directed to the arylamine compound as set forth in 1), in which R₁ to R₃ in the general formula (1) are the same or different, and each represent a hydrogen atom or a deuterium atom.
3) The present invention is further directed to the arylamine compound as set forth in 1) or 2), in which at least one of Ar₃ and Ar₄ in the general formula (1) represents a substituted or unsubstituted phenyl group, a substituted or unsubstituted biphenyl group, or a substituted or unsubstituted carbazolyl group.
4) The present invention is further directed to the arylamine compound as set forth in any one of 1) to 3), in which Ar₄ in the general formula (1) represents a substituted or unsubstituted phenyl group, a substituted or unsubstituted biphenyl group, or a substituted or unsubstituted carbazolyl group.
5) The present invention is further directed to the arylamine compound as set forth in any one of 1) to 4), in which L in the general formula (1) represents an unsubstituted phenylene group, an unsubstituted biphenylene group, or an unsubstituted naphthylene group.
6) The present invention is further directed to the arylamine compound as set forth in any one of 1) to 5), in which An and Ar₂ in the general formula (1) are the same or different, and each represent a substituted or unsubstituted phenyl group, a substituted or unsubstituted naphthyl group, a substituted or unsubstituted biphenyl group, a substituted or unsubstituted terphenyl group, a substituted or unsubstituted phenanthrenyl group, a substituted or unsubstituted triphenylsilyl group, a substituted or unsubstituted carbazolyl group, a substituted or unsubstituted dibenzofuranyl group, or a substituted or unsubstituted dibenzothienyl group.
7) The present invention is further directed to the arylamine compound as set forth in any one of 1) to 6), in which n in the general formula (1) is 1.
8) The present invention is further directed to an organic EL device having a pair of electrodes and one or more one organic layers sandwiched therebetween, in which at least one of the organic layers contains the arylamine compound as set forth in any one of 1) to 7).
9) The present invention is further directed to the organic EL device as set forth in 8), in which the organic layer is a hole-transporting layer.
10) The present invention is further directed to the organic EL device as set forth in 8), in which the organic layer is an electron-blocking layer.
11) The present invention is further directed to the organic EL device as set forth in 8), in which the organic layer is a hole-injecting layer.
12) The present invention is further directed to the organic EL device as set forth in 8), in which the organic layer is a light-emitting layer.
13) The present invention is further directed to an electronic apparatus including a device having a pair of electrodes and one or more organic layers sandwiched therebetween, in which at least one of the organic layers contains the arylamine compound as set forth in any one of 1) to 6).

Examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by An to Ar₄ in the general formula (1) include a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group, as well as an aryl group having 6 to 30 carbon atoms and a heteroaryl group having 2 to 20 carbon atoms.

Examples of the "substituent" in the "substituted aromatic hydrocarbon group", the "substituted aromatic heterocyclic group, or the "substituted fused polycyclic aromatic group" represented by An to Ar₄ in the general formula (1) include: a deuterium atom, a cyano group, and a nitro group; halogen atoms such as a fluorine atom, a chlorine atom, a bromine atom, and an iodine atom; silyl groups such as a trimethylsilyl group and a triphenylsilyl group; linear or branched alkyl groups having 1 to 6 carbon atoms such as a methyl group, an ethyl group, and a propyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups or fused polycyclic aromatic groups such as a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and aromatic heterocyclic groups such as a pyridyl group, a thienyl group, a furyl group, a pyrrolyl group, a quinolyl group, an isoquinolyl group, a benzofuranyl group, a benzothienyl group, an indolyl group, a carbazolyl group, a benzooxazolyl group, a benzothiazolyl group, a quinoxalinyl group, a benzimidazolyl group, a pyrazolyl group, a dibenzofuranyl group, a dibenzothienyl group, and a carbolinyl group. These substituents may further be substituted by any of the substituents listed above as examples. The substituent and the benzene ring substituted therewith, or a plurality of substituents on the same benzene ring may be bonded to each other to form a ring via a single bond, a substituted or unsubstituted methylene group, an oxygen atom, or a sulfur atom.

Examples of the "divalent aromatic hydrocarbon group", the "divalent aromatic heterocyclic group" or the "divalent fused polycyclic aromatic group" in the "substituted or unsubstituted divalent aromatic hydrocarbon group", the "substituted or unsubstituted divalent aromatic heterocyclic group", or the "substituted or unsubstituted divalent fused polycyclic aromatic group" represented by L in the general formula (1) include groups obtained by removing one hydrogen atom from those listed above as examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group" or the "substituted or unsubstituted fused polycyclic aromatic group" represented by An to Ar₄ in the general formula (1).

Examples of the "substituent" in the "substituted or unsubstituted divalent aromatic hydrocarbon group", the "substituted or unsubstituted divalent aromatic heterocyclic group", or the "substituted or unsubstituted divalent fused polycyclic aromatic group" represented by L in the general formula (1) include those listed above as examples of the "substituent" in the "substituted aromatic hydrocarbon group", the "substituted aromatic heterocyclic group", or the "substituted fused polycyclic aromatic group" represented by An to Ar₄ in the general formula (1), and the same holds true for the possible forms of these substituents.

Examples of the "linear or branched alkyl group having 1 to 6 carbon atoms", the "cycloalkyl group having 5 to 10 carbon atoms", or the "linear or branched alkenyl group having 2 to 6 carbon atoms" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₃ in the general formula (1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group. The substituent and the benzene ring substituted therewith, or a plurality of substituents on the same benzene ring may be bonded to each other to form a ring via a single bond, a substituted or unsubstituted methylene group, a substituted or unsubstituted amino group, an oxygen atom, or a sulfur atom.

Examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₃ in the general formula (1) include those listed above as examples of the "substituent" in the "substituted aromatic hydrocarbon group", the "substituted aromatic heterocyclic group", or the "substituted fused polycyclic aromatic group" represented by An to Ar₄ in the general formula (1), and the same holds true for the possible forms of these substituents.

Examples of the "linear or branched alkyloxy group having 1 to 6 carbon atoms" or the "cycloalkyloxy group having 5 to 10 carbon atoms" in the "linear or branched alkyloxy group having 1 to 6 carbon atoms and optionally having a substituent" or the "cycloalkyloxy group having 5 to 10 carbon atoms and optionally having a substituent" represented by R₁ to R₃ in the general formula (1) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. The substituent and the benzene ring substituted therewith, or a plurality of substituents on the same benzene ring may be bonded to each other to form a ring via a single bond, a substituted or unsubstituted methylene group, a substituted or unsubstituted amino group, an oxygen atom, or a sulfur atom.

Examples of the "substituent" in the "linear or branched alkyloxy group having 1 to 6 carbon atoms and optionally having a substituent" or the "cycloalkyloxy group having 5 to 10 carbon atoms and optionally having a substituent" represented by R₁ to R₃ in the general formula (1) include those listed above as examples of the "substituent" in the "substituted aromatic hydrocarbon group", the "substituted aromatic heterocyclic group", or the "substituted fused polycyclic aromatic group" represented by An to Ar₄ in the general formula (1), and the same holds true for the possible forms of these substituents.

Examples of the "aromatic hydrocarbon group", the "aromatic heterocyclic group", or the "fused polycyclic aromatic group" in the "substituted or unsubstituted aromatic hydrocarbon group", the " substituted or unsubstituted aromatic heterocyclic group", or the "substituted or unsubstituted fused polycyclic aromatic group" represented by R₁ to R₃ in the general formula (1) include those listed above as examples of the "aromatic hydrocarbon group", the "aromatic heterocyclic group", or the "fused polycyclic aromatic group in the "substituted or unsubstituted aromatic hydrocarbon group", the "substituted or unsubstituted aromatic heterocyclic group", or the "substituted or unsubstituted fused polycyclic aromatic group" represented by Ar₁ to Ar₄ in the general formula (1).

Examples of the "substituent" in the "substituted or unsubstituted aromatic hydrocarbon group", the "substituted or unsubstituted aromatic heterocyclic group", or the "substituted or unsubstituted fused polycyclic aromatic group" represented by R₁ to R₃ in the general formula (1) include those listed above as examples of the "substituent" in the "substituted aromatic hydrocarbon group", the "substituted aromatic heterocyclic group", or the "substituted fused polycyclic aromatic group" represented by An to Ar₄ in the general formula (1), and the same holds true for the possible forms of these substituents.

Examples of the "aryloxy group" in the "substituted or unsubstituted aryloxy group" represented by R₁ to R₃ in the general formula (1) include a phenyloxy group, a biphenylyloxy group, a terphenylyloxy group, a naphthyloxy group, an anthracenyloxy group, a phenanthrenyloxy group, a fluorenyloxy group, a spirobifluorenyloxy group, an indenyloxy group, a pyrenyloxy group, a perylenyloxy group, a fluoranthenyloxy group, a triphenylenyloxy group, a benzofuranyloxy group, a benzothienyloxy group, an indolyloxy group, a carbazolyloxy group, a dibenzofuranyloxy group, and a dibenzothienyloxy group. Other examples include an aryl group having 6 to 30 carbon atoms and a heteroaryl group having 2 to 20 carbon atoms. A substituent and the benzene ring substituted therewith, or a plurality of substituents on the same benzene ring may be bonded to each other to form a ring via a single bond, a substituted or unsubstituted methylene group, a substituted or unsubstituted amino group, an oxygen atom, or a sulfur atom.

Examples of the "substituent" in the "substituted or unsubstituted aryloxy group" represented by R₁ to R₃ in the general formula (1) include those listed above as examples of the "substituent" in the substituted aromatic hydrocarbon group", the "substituted aromatic heterocyclic group", or the "substituted fused polycyclic aromatic group" represented by Ar₁ to Ar₄ in the general formula (1), and the same holds true for the possible forms of these substituents.

At least one of Ar₃ and Ar₄ in the general formula (1) preferably represents a substituted or unsubstituted phenyl group, a substituted or unsubstituted biphenyl group, or a substituted or unsubstituted carbazolyl group, and more preferably represents an unsubstituted phenyl group, an unsubstituted biphenyl group, or an unsubstituted carbazolyl group.

Ar₃ and Ar₄ in the general formula (1) are the same or different, and each preferably represent a substituted or unsubstituted phenyl group, a substituted or unsubstituted biphenyl group, or a substituted or unsubstituted naphthyl group in view of excellent electron-blocking capability, each more preferably represent an unsubstituted phenyl group, an unsubstituted biphenyl group, or an unsubstituted naphthyl group, and each even more preferably represent an unsubstituted phenyl group or an unsubstituted naphthyl group.

In the general formula (1), L preferably represents an unsubstituted phenylene group, an unsubstituted biphenylene group, or an unsubstituted naphthylene group in view of excellent electron-blocking capability, and more preferably represents an unsubstituted phenylene group or an unsubstituted biphenylene group.

In the general formula (1), n is preferably 1.

In the general formula (1), An and Ar₂ are the same or different, and each preferably represent a substituted or unsubstituted phenyl group, a substituted or unsubstituted biphenyl group, a substituted or unsubstituted naphthyl group, a substituted or unsubstituted phenanthrenyl group, or a substituted or unsubstituted terphenyl group in view of excellent electron-blocking capability, and each more preferably represent a substituted or unsubstituted phenyl group, a substituted or unsubstituted biphenyl group, or a substituted or unsubstituted terphenyl group.

In the general formula (1), R₁ to R₃ are the same or different, and each preferably represent a hydrogen atom or a deuterium atom, and each more preferably represent a hydrogen atom in view of ease of synthesis.

The arylamine compound represented by the general formula (1), which is suitable for use in an organic EL device of the present invention, is preferably used as a material for forming a hole-injecting layer, a hole-transporting layer, an electron-blocking layer, or a light-emitting layer of the organic EL device, and more preferably used as a material for forming the hole-transporting layer or the electron-blocking layer.

The arylamine compound of the present invention has the following properties: (1) better hole-injecting performance, (2) higher hole mobility, (3) excellent electron-blocking capability, (4) higher electron resistance, (5) higher stability in the form of a film, and (6) excellent heat resistance, compared with those of conventional materials. When the arylamine compound of the present invention is used for an organic EL device, the resulting organic EL device has the following properties: (7) high luminous efficacy, (8) a low voltage for the start of light emission, (9) a low actual driving voltage, and (10) a long lifespan.

The arylamine compound of the present invention is excellent in terms of hole-injecting/transporting performance, stability in the form of a film, and durability. Accordingly, the organic EL device having a hole-injecting layer and/or a hole-transporting layer formed by using this compound as a hole injection material and/or a hole transport material has higher efficiency in transporting holes to the light-emitting layer, and therefore higher luminous efficacy, and further has a lower driving voltage, and therefore improved device durability. Thus, the device has improved properties including high efficiency, a low driving voltage, and a long lifespan.

The arylamine compound of the present invention has excellent electron-blocking capability, high electron resistance, and high stability in the form of a film, and can confine excitons generated in the light-emitting layer. Thus, the organic EL device having an electron-blocking layer formed by using this compound as an electron-blocking material has an increased probability of recombinations of holes and electrons to prevent thermal deactivation, and therefore has a higher luminous efficacy, and the organic EL device also has a lower driving voltage and therefore higher current resistance, and accordingly, has an increased maximum luminance.

The arylamine compound of the present invention has excellent hole-transporting capability and a wide bandgap. Accordingly, the organic EL device having a light-emitting layer formed by using this compound as a host material and doping thereinto a so-called dopant, such as a fluorescent emitter, a phosphorescent emitter, or a delayed fluorescent emitter has a lower driving voltage and a higher luminous efficacy.

Accordingly, the arylamine compound of the present invention is useful as materials of a hole-injecting layer, a hole-transporting layer, an electron-blocking layer, or a light-emitting layer of an organic EL device, and can improve the luminous efficacy, the driving voltage, and the durability, compared with those of conventional organic EL devices.

Moreover, the arylamine compound of the present invention can be applied not only to organic EL devices but also in the fields of electronic apparatuses, specifically, to photoreceptors for electrophotography, image sensors, photoelectric transducers, solar cells, and the like.

### Brief Description of Drawings

[Fig. 1] Fig. 1 shows Compounds (1) to (12) as preferred specific examples of the arylamine compound represented by the general formula (1).
[Fig. 2] Fig. 2 shows Compounds (13) to (24) as preferred specific examples of the arylamine compound represented by the general formula (1).
[Fig. 3] Fig. 3 shows Compounds (25) to (36) as preferred specific examples of the arylamine compound represented by the general formula (1).
[Fig. 4] Fig. 4 shows Compounds (37) to (48) as preferred specific examples of the arylamine compound represented by the general formula (1).
[Fig. 5] Fig. 5 shows Compounds (49) to (60) as preferred specific examples of the arylamine compound represented by the general formula (1).
[Fig. 6] Fig. 6 shows Compounds (61) to (75) as preferred specific examples of the arylamine compound represented by the general formula (1).
[Fig. 7] Fig. 7 shows Compounds (76) to (87) as preferred specific examples of the arylamine compound represented by the general formula (1).
[Fig. 8] Fig. 8 shows Compounds (88) to (99) as preferred specific examples of the arylamine compound represented by the general formula (1).
[Fig. 9] Fig. 9 shows Compounds (100) to (111) as preferred specific examples of the arylamine compound represented by the general formula (1).
[Fig. 10]Fig. 10 shows Compounds (112) to (123) as preferred specific examples of the arylamine compound represented by the general formula (1).
[Fig. 11]Fig. 11 shows Compounds (124) to (135) as preferred specific examples of the arylamine compound represented by the general formula (1).
[Fig. 12]Fig. 12 shows Compounds (136) to (146) as preferred specific examples of the arylamine compound represented by the general formula (1).
[Fig. 13]Fig. 13 shows Compounds (147) to (158) as preferred specific examples of the arylamine compound represented by the general formula (1).
[Fig. 14]Fig. 14 shows Compounds (159) to (170) as preferred specific examples of the arylamine compound represented by the general formula (1).
[Fig. 15]Fig. 15 shows Compounds (171) to (182) as preferred specific examples of the arylamine compound represented by the general formula (1).
[Fig. 16]Fig. 16 shows Compounds (183) to (185) as preferred specific examples of the arylamine compound represented by the general formula (1).
[Fig. 17]Fig. 17 is a diagram showing the configuration of an organic EL device produced in each of Examples 17 to 21 and Comparative Examples 1 and 2.

### Description of Embodiments

The arylamine compound of the present invention is a novel compound. The compound can be synthesized according to methods known per se (see Patent Literature 5, for example).

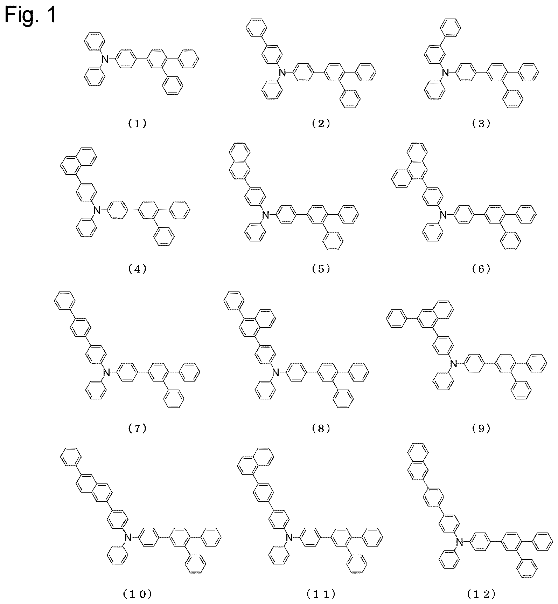

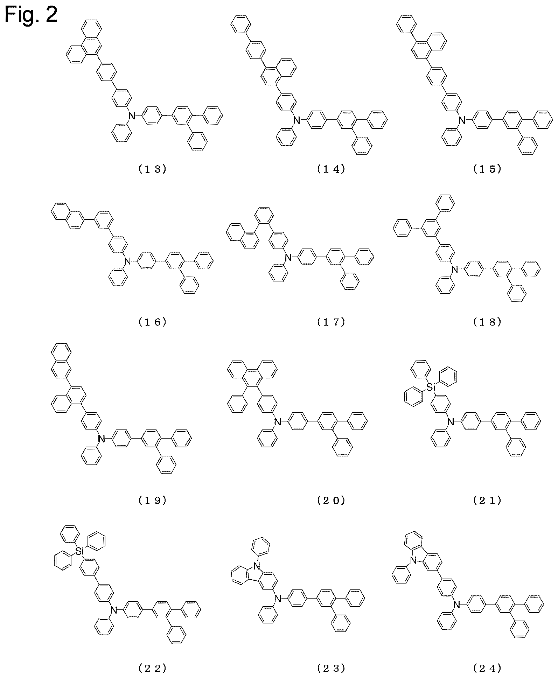

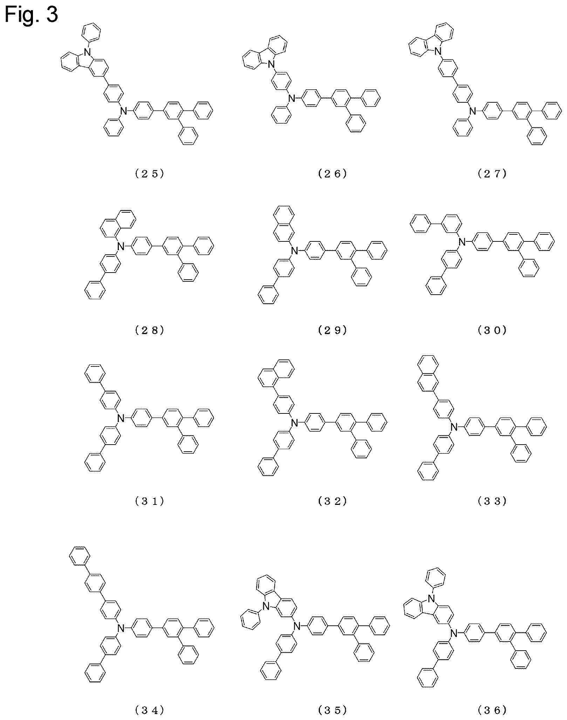

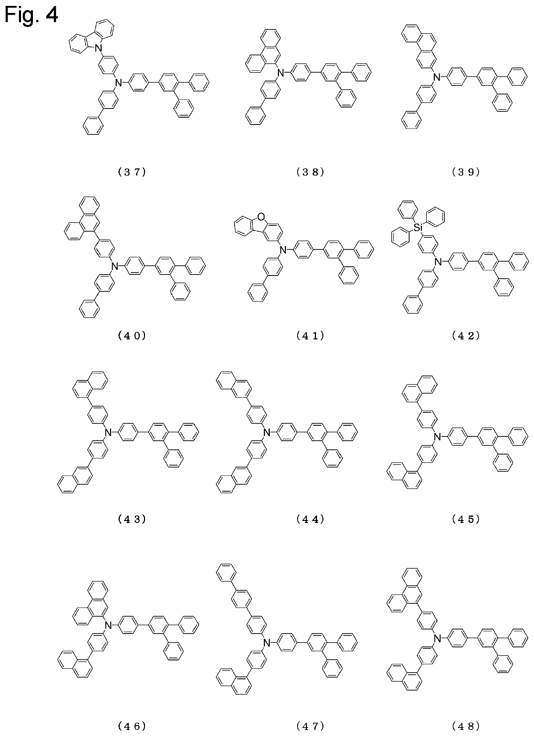

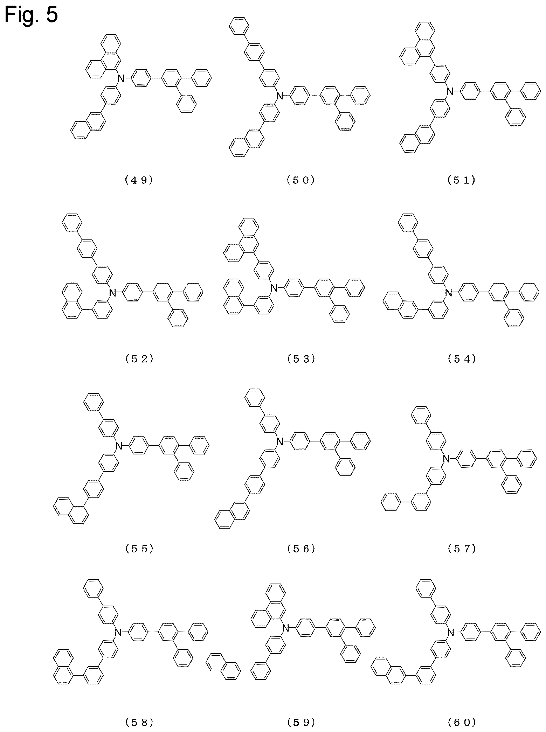

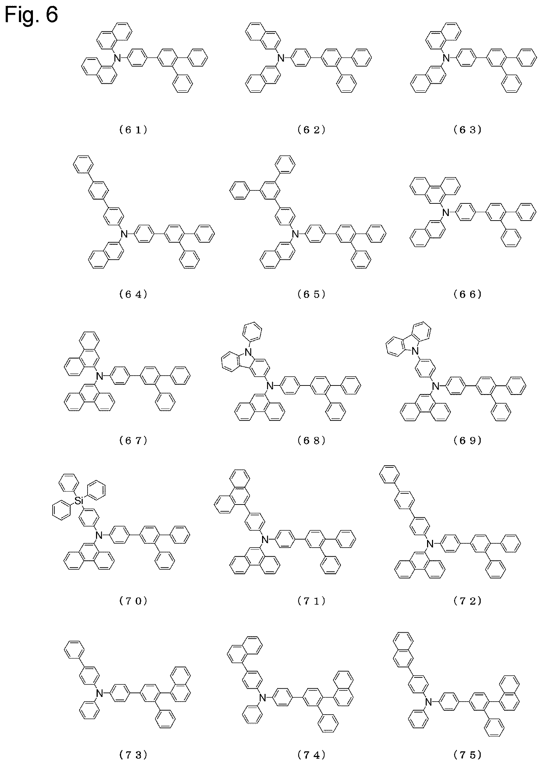

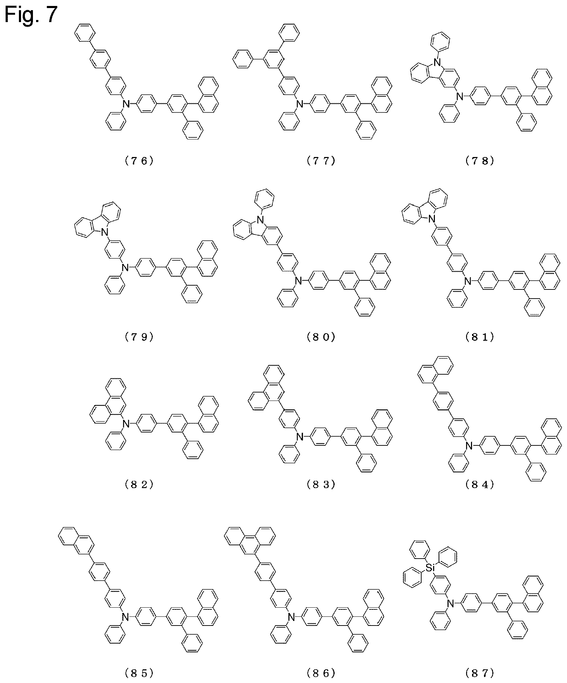

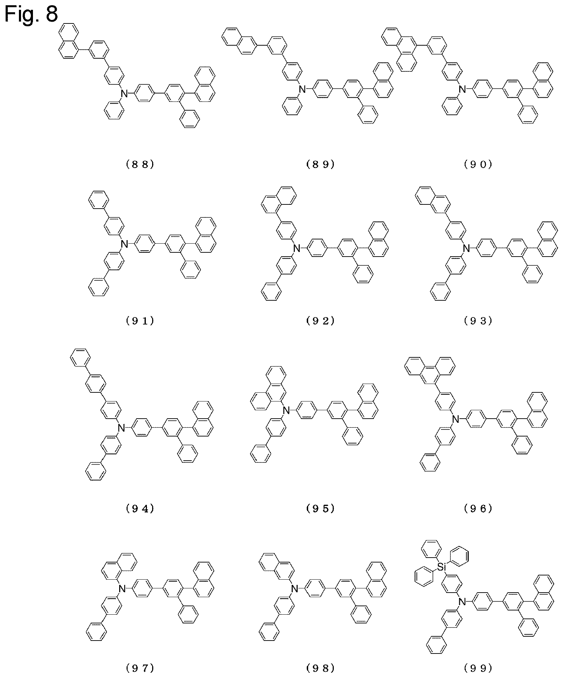

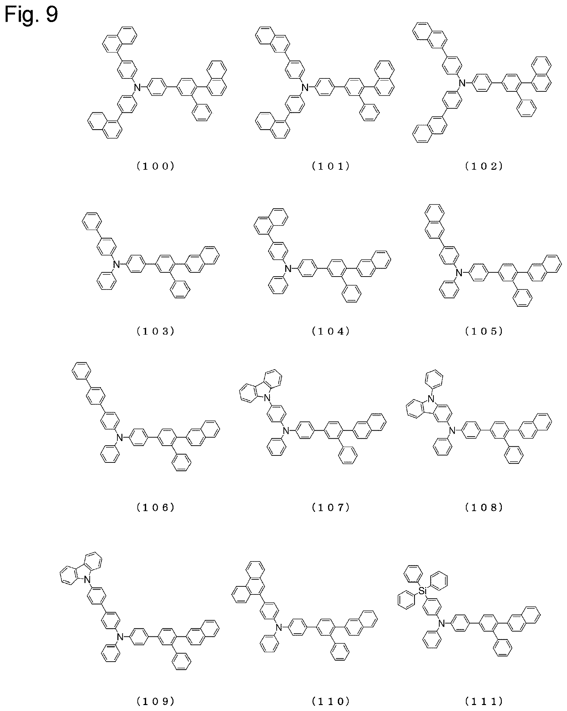

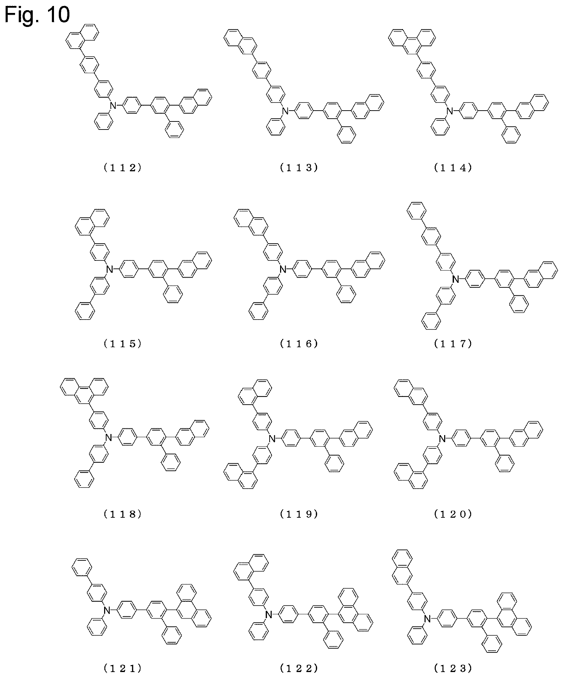

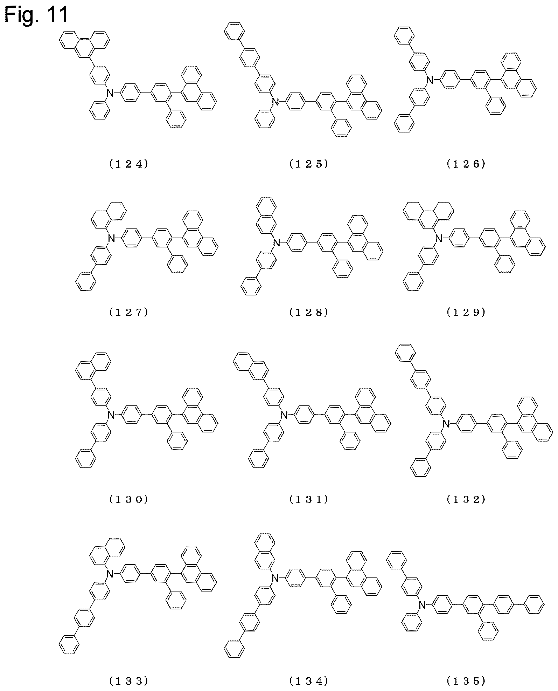

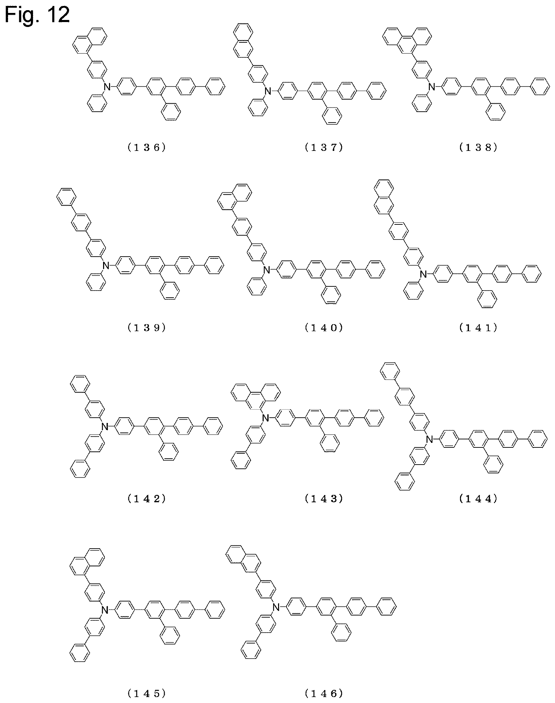

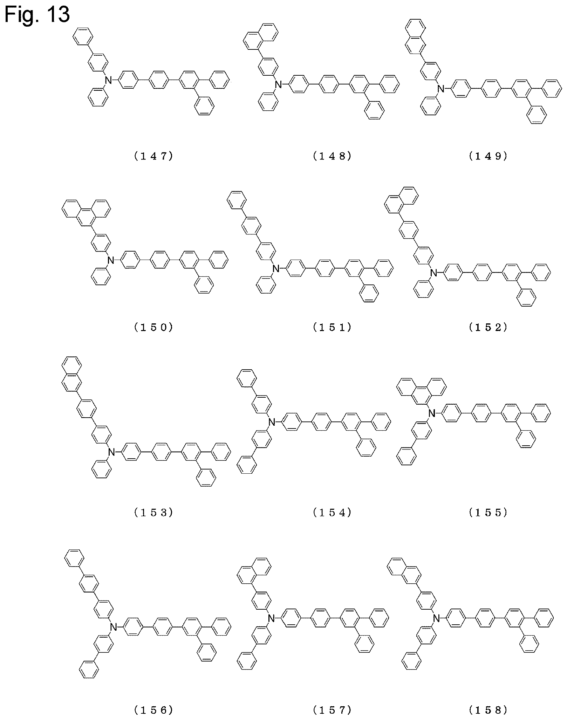

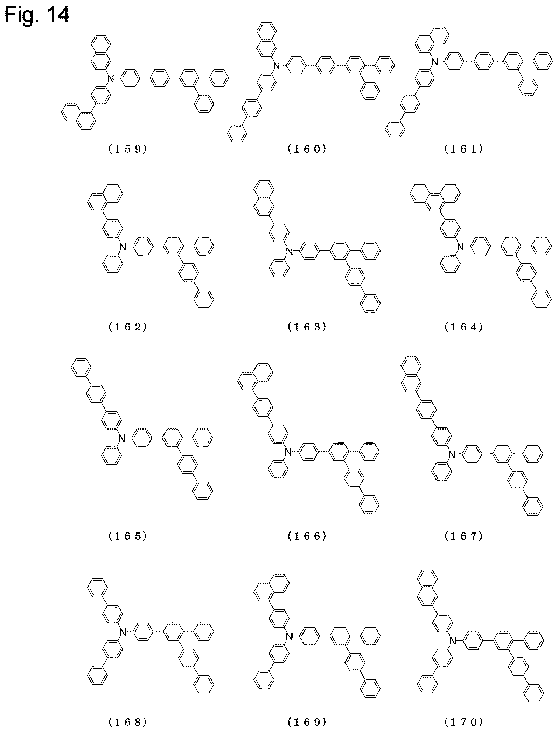

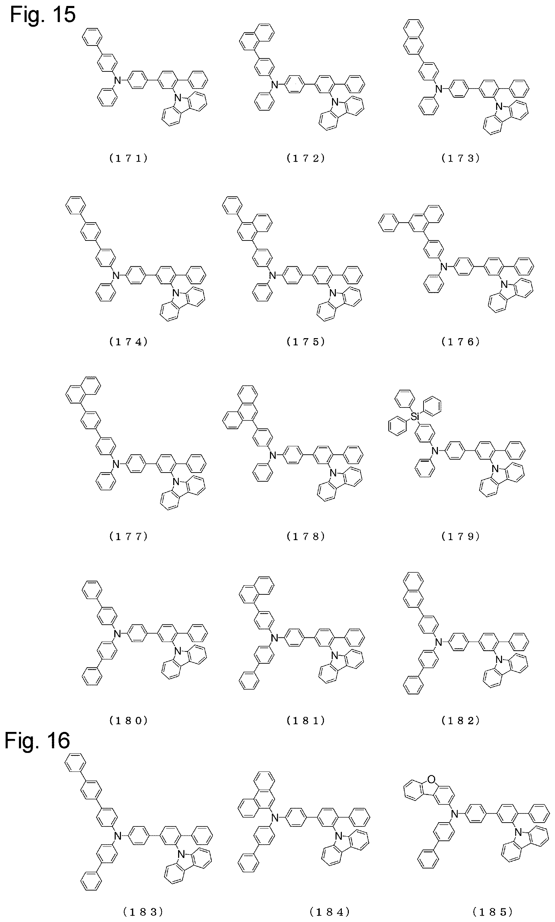

Specific preferred examples of the arylamine compounds represented by the general formula (1), which are suitably used for the organic EL device of the present invention, are shown in Figs. 1 to 16. However, the present invention is not limited to these compounds.

The arylamine compound represented by the general formula (1) can be purified by any known method, such as column chromatography, adsorption with silica gel, activated carbon, activated clay, or the like, recrystallization or crystallization from a solvent, or sublimation. The compound can be identified by NMR analysis. The physical properties can be determined in terms of the melting point, the glass transition point (Tg), and the work function, and others. The melting point is a measure of the vapor deposition properties. The glass transition point (Tg) is a measure of the stability in the form of a film. The work function is a measure of the hole-injecting capability, the hole-transporting capability, and the electron-blocking capability.

The melting point and the glass transition point (Tg) can be measured, for example, on the compound in the form of a powder using a high-sensitivity differential scanning calorimeter (DSC3100SA manufactured by Bruker AXS K.K.).

The work function can be determined, for example, on the compound in the form of a thin film with a thickness of 100 nm on an ITO substrate using an ionization potential measurement system (PYS-202 manufactured by Sumitomo Heavy Industries, Ltd.).

The organic EL device of the present invention may have: a structure in which an anode, a hole-injecting layer, a hole-transporting layer, a light-emitting layer, an electron-transporting layer, an electron-injecting layer, and a cathode are sequentially formed on a substrate; and the structure may further include an electron-blocking layer between the hole-transporting layer and the light-emitting layer and/or a hole-blocking layer between the light-emitting layer and the electron-transporting layer. In these multilayer structures, a single organic layer can perform the functions of several layers. For example, a single organic layer may serve as both the hole-injecting layer and the hole-transporting layer, and a single organic layer may serve as both the electron-injecting layer and the electron-transporting layer. It is also possible to stack two or more organic layers having the same function. Specifically, two hole-transporting layers may be stacked; two light-emitting layers may be stacked; and two electron-transporting layers may be stacked.

An electrode material having a high work function, such as ITO or gold, is used for the anode of the organic EL device of the present invention. Examples of the materials of the hole-injecting layer of the organic EL device of the present invention include porphyrin compounds typified by copper phthalocyanine; starburst triphenylamine derivatives; arylamine compounds having a structure containing two or more triphenylamine or carbazolyl structures in the molecule, the triphenylamine or carbazolyl structures being linked via a single bond or a divalent group having no heteroatom; acceptor type heterocyclic compounds such as hexacyanoazatriphenylene; and coating type polymer materials. These materials can be formed into a thin film using a known method such as vapor deposition, spin coating, or inkjet printing.

Examples of the materials used for the hole-injecting layer and the hole-transporting layer of the organic EL device of the present invention include: the arylamine compound of the present invention; benzidine derivatives such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylyl benzidine; 1,1-bis[(di-4-tolylamino)phenyl]cyclohexane (TAPC); and arylamine compounds having a structure containing two or more triphenylamine or carbazolyl structures in the molecule, the triphenylamine or carbazolyl structures being linked via a single bond or a divalent group having no heteroatom. These materials may be used singly for film formation, or two or more of these materials may be mixed and used for film formation. In each case, a single layer may be formed. The hole-injecting layer and the hole-transporting layer each may have a layered structure composed of different layers each formed of a single kind of the materials described above; a layered structure composed of different layers each formed of a mixture of any materials described above; or a layered structure composed of a layer formed of a single kind of the materials described above and a layer formed of a mixture of two or more of the materials described above. Other examples of the materials of the hole-inj ecting/transporting layers include coating type polymer materials such as poly(3,4-ethylenedioxythiophene) (PEDOT)/poly(styrenesulfonate) (PSS). These materials can be formed into a thin film using a known method such as vapor deposition, spin coating, or inkjet printing.

Other examples of the materials used for the hole-injecting layer or the hole-transporting layer include a material obtained by p-doping a material normally used for these layers with trisbromophenylamine hexachloroantimony or a radialene derivative (see Patent Literature 6, for example); and a polymer compound having the structure of a benzidine derivative, such as TPD, as a partial structure thereof.

Examples of the materials for the electron-blocking layer of the organic EL device of the present invention include: the arylamine compound of the present invention; and also compounds having an electron-blocking effect, such as carbazole derivatives such as 4,4',4"-tri(N-carbazolyl)triphenylamine (TCTA), 9,9-bis[4-(carbazole-9-yl)phenyl]fluorene, 1,3-bis(carbazole-9-yl)benzene (mCP), and 2,2-bis(4-carbazole-9-ylphenyl)adamantane (Ad-Cz), and compounds having a triphenylsilyl group and a triarylamine structure such as 9-[4-(carbazole-9-yl)phenyl]-9-[4-(triphenylsilyl)phenyl]-9H-fluorene. These materials may also serve as the material for the hole-transporting layer. These materials may be used singly for film formation, or two or more of these materials may be mixed and used for film formation. In each case, a single layer may be formed. The electron-blocking layer may have a layered structure composed of different layers each formed of a single kind of the materials described above; a layered structure composed of different layers each formed of a mixture of any materials described above; or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of the materials for the light-emitting layer of the organic EL device of the present invention include: the arylamine compound of the present invention; metal complexes of quinolinol derivatives such as tris(8-quinolinato)aluminum (Alq₃); various types of metal complexes; an anthracene derivative; a bisstyrylbenzene derivative; a pyrene derivative; an oxazole derivative; and a polyparaphenylene vinylene derivative. The light-emitting layer may also include a host material and a dopant material. As the host material, an anthracene derivative is preferably used. Other examples of the host material include: the light emitting materials including the arylamine compound of the present invention; and also a heterocyclic compound having an indole ring as a partial structure of a fused ring, a heterocyclic compound having a carbazole ring as a partial structure of a fused ring, a carbazole derivative, a thiazole derivative, a benzimidazole derivative, and a polydialkylfluorene derivative. Examples of the dopant material include quinacridone, coumalin, rubrene, perylene, and derivatives thereof; a benzopyran derivative; a rhodamine derivative; and an aminostyryl derivative. These materials may be used singly for film formation, or two or more of these materials may be mixed and used for film formation. In each case, a single layer may be formed. The light-emitting layer may have a layered structure composed of different layers each formed of a single kind of the materials described above; a layered structure composed of different layers each formed of a mixture of any materials described above; or a layered structure composed of a layer formed of a single kind of the materials described above and a layer formed of a mixture of two or more of the materials described above. These material can be formed into a thin film using a known method such as vapor deposition, spin coating, or inkjet printing.

A phosphorescent emitter can also be used as a light-emitting material. The phosphorescent emitter may be a metal complex of iridium, platinum, or the like, and examples thereof include a green phosphorescent emitter such as Ir(ppy)₃, a blue phosphorescent emitter such as FIrpic or FIr6, and a red phosphorescent emitter such as Btp₂Ir (acac). As a host material for this case, a host material having hole-injecting/transporting capability may be used, including carbazole derivatives such as 4,4'-di(N-carbazolyl)biphenyl (CBP), TCTA, and mCP, and also the arylamine compound of the present invention. A host material having electron-transporting capability may also be used, including p-bis(triphenylsilyl)benzene (UGH2) and 2,2',2"-(1,3,5-phenylene)-tris(1-phenyl-1H-benzimidazole) (TPBI). Use of these materials enables production of a high-performance organic EL device.

In order to avoid concentration quenching, the doping of a host material with a phosphorescent emitter is preferably performed by co-deposition in an amount within a range of 1 to 30 wt% based on the entire light-emitting layer.

As the light emitting material, a material that emits delayed fluorescence can also be used, including a CDCB derivative, specifically, PIC-TRZ, CC2TA, PXZ-TRZ, and 4CzIPN (see Non-Patent Literature 3, for example). These materials can be formed into a thin film using a known method such as vapor deposition, spin coating, or inkjet printing.

Examples of the materials for the hole-blocking layer of the organic EL device of the present invention include compounds having a hole-blocking effect, including a phenanthroline derivative such as bathocuproine (BCP); a metal complex of a quinolinol derivative such as bis(2-methyl-8-quinolinolate)-4-(phenylphenolate)aluminum (BAlq); various types of rare-earth complexes; an oxazole derivative; a triazole derivative; and a triazine derivative. These materials may also serve as the material for the electron-transporting layer. These materials may be used singly for film formation, or two or more of these materials may be mixed and used for film formation. In each case, a single layer may be formed. The hole-blocking layer may have a layered structure composed of different layers each formed of a single kind of the materials described above; a layered structure composed of different layers each formed of a mixture of any materials described above; or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of the materials for the electron-transporting layer of the organic EL device of the present invention include: metal complexes of quinolinol derivatives, such as Alq₃ and BAlq; various types of metal complexes; a triazole derivative; a triazine derivative; an oxadiazole derivative; a pyridine derivative; a pyrimidine derivative; a benzimidazole derivative; a thiadiazole derivative; an anthracene derivative; a carbodiimide derivative; a quinoxaline derivative; a pyridoindole derivative; a phenanthroline derivative; and a silole derivative. These materials may be used singly for film formation, or two or more of these materials may be mixed and used for film formation. In each case, a single layer may be formed. The electron-transporting layer may have a layered structure composed of different layers each formed of a single kind of the materials described above; a layered structure composed of different layers each formed of a mixture of any materials described above; or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of the materials for the electron-injecting layer of the organic EL device of the present invention include: alkali metal salts such as lithium fluoride and cesium fluoride; alkaline earth metal salts such as magnesium fluoride; metal complexes of quinolinol derivatives such as lithium quinolinol; metal oxides such as aluminum oxide; and metals such as ytterbium (Yb), samarium (Sm), calcium (Ca), strontium (Sr), and cesium (Cs). The electron-injecting layer can however be omitted when an electron-transporting layer and a cathode are suitably selected.

Furthermore, a material obtained by n-doping a material normally used for an electron-injecting layer or an electron-transporting layer with a metal such as cesium can be used for the electron-injecting layer and the electron-transporting layer.

For the cathode of the organic EL device of the present invention, a metal having a low work function, such as aluminum; or an alloy having an even lower work function, such as a magnesium-silver alloy, a magnesium-indium alloy, or an aluminum-magnesium alloy is used as the electrode material.

### Examples

Hereinafter, embodiments of the present invention will be described in greater detail by way of examples. However, the present invention is not limited to the examples below, without departing from the spirit thereof.

### Example 1

### <Synthesis of biphenyl-4-yl-phenyl-(2'-phenyl-[1,1':4',1"]terphenyl-4"-yl)-amine (Compound (2))>

First, 49.0 g of 4'-iodo-[1,1':2',1"]terphenyl, 55.3 g of biphenyl-4-yl-phenyl-amino-4-phenylboronic acid, 4.0 g of tetrakis(triphenylphosphine)palladium(0), and 28.5 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through column chromatography (support: silica gel, eluent: toluene/n-heptane) to obtain 65.7 g of a pale yellow powder of biphenyl-4-yl-phenyl-(2'-phenyl-[1,1':4',1"]terphenyl-4"-yl)-amine (Compound (2), yield: 86.9%).

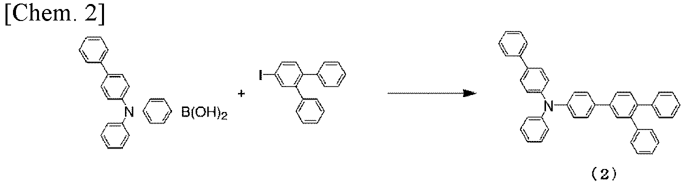

### Example 2

### <Synthesis of biphenyl-4-yl-{4-(naphthalene-2-yl)-phenyl}-(2'-phenyl-[1,1':4',1"]terphenyl-4"-yl)-amine (Compound (33))>

First, 14.7 g of biphenyl-4-yl-{4-(naphthalene-2-yl)-phenyl}-phenyl-amine was placed in a reaction vessel, and stirred in a dichloromethane solvent while icing. Then 5.9 g of N-bromosuccinimide was added thereto, and the resultant was stirred at room temperature overnight. After the end of the reaction, H₂O was added to the system, followed by extraction and partition to obtain the organic layer, and the organic layer was concentrated to give a crude product. The crude product was purified through recrystallization from an acetone solvent to obtain 16.7 g of a white powder of biphenyl-4-yl-(4-bromophenyl)-{4-(naphthalene-2-yl)phenyl}-amine (yield: 96.6%).

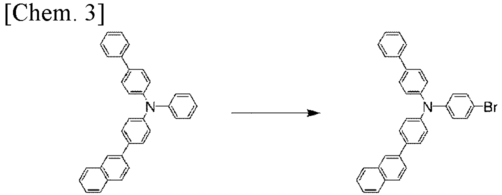

Next, 9.0 g of biphenyl-4-yl-(4-bromophenyl)-{4-(naphthalene-2-yl)phenyl}-amine, 6.7 g of 4,4,5,5-tetramethyl-2-[1,1':2',1]terphenyl-4'-yl-[1,3,2]dioxaborolane, 0.5 g of tetrakis(triphenylphosphine)palladium(0), and 3.1 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from a toluene/acetone mixed solvent to obtain 7.2 g of a white powder of biphenyl-4-yl-{4-(naphthalene-2-yl)-phenyl}-(2'-phenyl-[1,1':4',1"]terphenyl-4"-yl)-amine (Compound (33), yield: 62.3%).

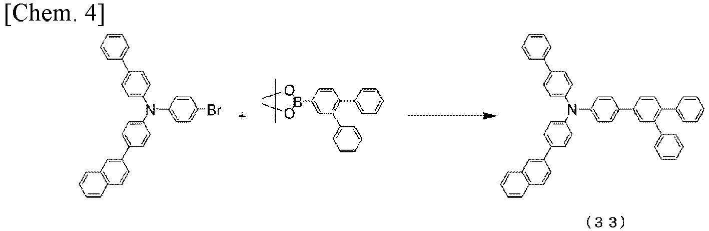

The structure of the obtained white powder was identified using NMR.

The following 37 hydrogen signals were detected using ¹H-NMR (CDCl₃). δ (ppm) = 8.07 (1H), 7.93 (2H), 7.89 (1H), 7.79 (1H), 7.70 (3H), 7.69 (1H), 7.65 (2H), 7.63 (2H), 7.58 (2H), 7.52 (3H), 7.47 (2H), 7.39-7.18 (17H).

### Example 3

### <Synthesis of biphenyl-4-yl-(2'-phenyl-[1,1':4',1"]terphenyl-4"-yl)-([1,1':4',1"]terphenyl-4"-yl)-amine (Compound (34))>

9.0 g of biphenyl-4-yl-phenyl-(2'-phenyl-[1,1':4',1"]terphenyl-4"-yl)-amine (Compound (2)) was placed in a reaction vessel, and stirred in a dichloromethane solvent while icing. Then 21.0 g of N-bromosuccinimide was added thereto, and the resultant was stirred at room temperature overnight. After the end of the reaction, H₂O was added to the system, followed by extraction and partition to obtain the organic layer, and the organic layer was concentrated to give a crude product. The crude product was purified through crystallization from an acetone/n-heptane mixed solvent to obtain 64.0 g of a white powder of biphenyl-4-yl-(4-bromophenyl)-(2'-phenyl-[1,1':4',1"]terphenyl-4"-yl)-amine (yield: 85.4%).

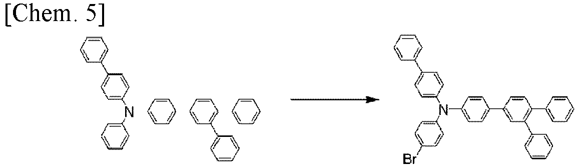

Next, 9.0 g of biphenyl-4-yl-(4-bromophenyl)-(2'-phenyl-[1,1':4',1"]terphenyl-4"-yl)-amine, 3.1 g of 4-biphenylboronic acid, 0.4 g of tetrakis(triphenylphosphine)palladium(0), and 2.6 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from a toluene/acetone mixed solvent to obtain 9.5 g of a white powder of biphenyl-4-yl-(2'-phenyl-[1,1':4',1"]terphenyl-4"-yl)-([1,1':4',1"]terphenyl-4"-yl)-amine (Compound (34), yield: 94.5%).

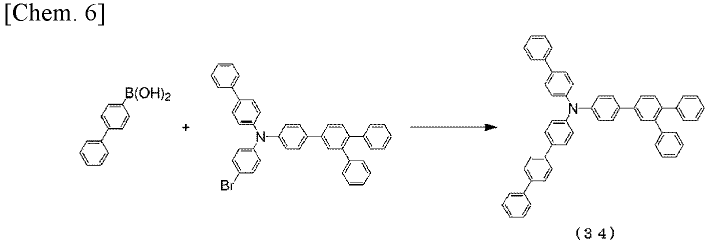

The structure of the obtained white powder was identified using NMR.

The following 39 hydrogen signals were detected using ¹H-NMR (CDCl₃).

δ (ppm) = 7.71 (2H), 7.70 (4H), 7.66 (3H), 7.62 (3H), 7.59 (2H), 7.56 (1H), 7.53 (1H), 7.48 (4H), 7.39 (1H), 7.35 (1H), 7.30 (3H), 7.28 (5H), 7.27-7.19 (9H).

### Example 4

### <Synthesis of biphenyl-4-yl- { 4-(phenanthrene-9-yl)-phenyl} -(2 ' -phenyl-[1,1':4',1"]terphenyl-4"-yl)-amine (Compound (40))>

9.0 g of biphenyl-4-yl-(4-bromophenyl)-(2'-phenyl-[1,1':4',1"]terphenyl-4"-yl)-amine, 3.5 g of 9-phenanthreneboronic acid, 0.4 g of tetrakis(triphenylphosphine)palladium(0), and 2.6 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from a toluene/acetone mixed solvent to obtain 8.4 g of a white powder of biphenyl-4-yl-{4-(phenanthrene-9-yl)-phenyl}-(2'-phenyl-[1,1':4',1"]terphenyl-4"-yl)-amine (Compound (40), yield: 80.8%).

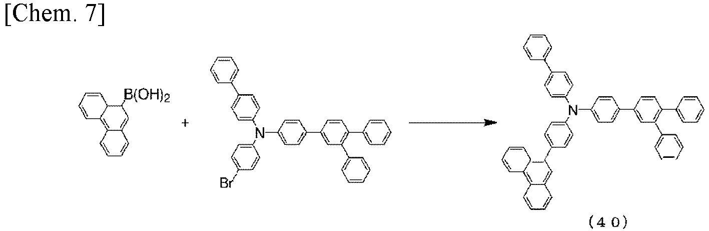

The structure of the obtained white powder was identified using NMR.

The following 39 hydrogen signals were detected using ¹H-NMR (CDCl₃).

δ (ppm) = 8.79 (1H), 8.73 (1H), 8.08 (1H), 7.91 (1H), 7.74 (1H), 7.73-7.54 (12H), 7.51 (1H), 7.48 (2H), 7.44 (2H), 7.33 (7H), 7.25-7.16 (10H).

### Example 5

### <Synthesis of {4-(naphthalene-1-yl)-phenyl}-{4-(naphthalene-2-yl)-phenyl}-(2'-phenyl-[1,1':4',1"]terphenyl-4"-yl)-amine (Compound (43))>

9.0 g of (4-bromophenyl)-{4-(naphthalene-1-yl)-phenyl}-{4-(naphthalene-2-yl)-phenyl}-amine, 6.1 g of 4,4,5,5-tetramethyl-2-[1,1':2',1]terphenyl-4'-yl-[1,3,2]dioxaborolane, 0.5 g of tetrakis(triphenylphosphine)palladium(0), and 2.8 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through column chromatography (support: silica gel, eluent: dichloromethane/n-heptane) to obtain 9.3 g of a white powder of {4-(naphthalene-1-yl)-phenyl}-{4-(naphthalene-2-yl)-phenyl}-(2'-phenyl-[1,1':4',1"]terphenyl-4"-yl)-amine (Compound (43), yield: 82.1%).

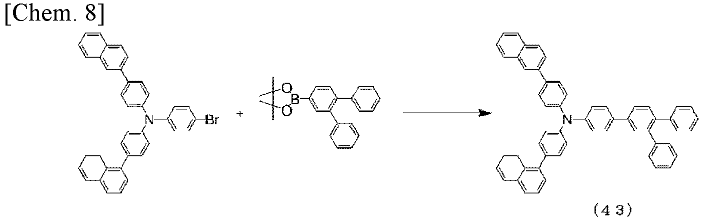

The structure of the obtained white powder was identified using NMR.

The following 39 hydrogen signals were detected using ¹H-NMR (CDCl₃).

δ (ppm) = 8.05 (2H), 7.91 (3H), 7.86 (2H), 7.78 (1H), 7.70 (2H), 7.68 (2H), 7.65 (2H), 7.57-7.42 (9H), 7.36 (3H), 7.33 (3H), 7.25-7.16 (10H).

### Example 6

### <Synthesis of bis{4-(naphthalene-1-yl)-phenyl}-(2'-phenyl-[1,1':4',1"]terphenyl-4"-yl)-amine (Compound (45))>

9.0 g of bis{4-(naphthalene-2-yl)phenyl}-(4-bromophenyl)-amine, 6.1 g of 4,4,5,5-tetramethyl-2-[1,1':2',1]terphenyl-4'-yl-[1,3,2]dioxaborolane, 0.5 g of tetrakis(triphenylphosphine)palladium(0), and 2.8 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through column chromatography (support: silica gel, eluent: dichloromethane/n-heptane) to obtain 7.6 g of a white powder of bis{4-(naphthalene-1-yl)-phenyl}-(2'-phenyl-[1,1':4',1 "]terphenyl-4"-yl)-amine (Compound (45), yield: 67.1%).

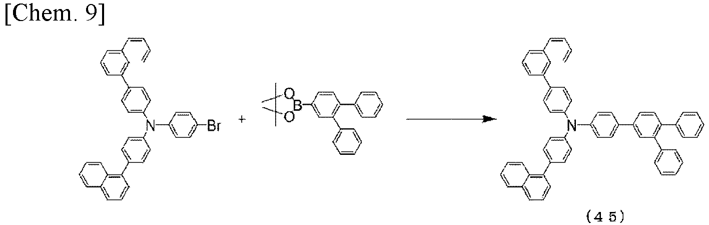

The structure of the obtained white powder was identified using NMR.

The following 39 hydrogen signals were detected using ¹H-NMR (CDCl₃).

δ (ppm) = 8.07 (2H), 7.92 (2H), 7.86 (2H), 7.68 (2H), 7.66 (1H), 7.65 (1H), 7.57-7.44 (13H), 7.40-7.34 (6H), 7.25-7.17 (10H).

### Example 7

### <Synthesis of biphenyl-4-yl-{4'-(naphthalene-1-yl)-biphenyl-4-yl}-(2'-phenyl-[1,1':4',1"]terphenyl-4"-yl)-amine (Compound (55))>

9.0 g of biphenyl-4-yl-(4-bromophenyl)-(2'-phenyl-[1,1':4',1"]terphenyl-4"-yl)-amine, 3.9 g of 4-(naphthalene-1-yl)phenylboronic acid, 0.4 g of tetrakis(triphenylphosphine)palladium(0), 2.6 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from a toluene/acetone mixed solvent to obtain 9.6 g of a white powder of biphenyl-4-yl- { 4'-(naphthalene-1-yl)-biphenyl-4-yl}-(2'-phenyl-[1,1':4',1"]terphenyl-4"-yl)-amine (Compound (55), yield: 89.1%).

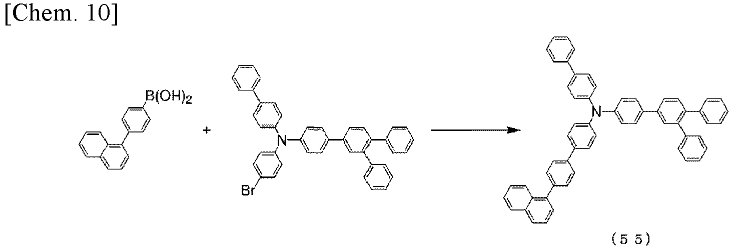

The structure of the obtained white powder was identified using NMR.

The following 41 hydrogen signals were detected using ¹H-NMR (CDCl₃).

δ (ppm) = 7.99 (1H), 7.92 (1H), 7.88 (1H), 7.73 (2H), 7.67 (1H), 7.66-7.59 (7H), 7.56 (5H), 7.53 (1H), 7.51 (1H), 7.49 (1H), 7.47 (2H), 7.43 (2H), 7.34 (1H), 7.29 (6H), 7.25-7.16 (9H).

### Example 8

### <Synthesis of biphenyl-4-yl-{3'-(naphthalene-1-yl)-biphenyl-4-yl}-(2'-phenyl-[1,1':4',1"]terphenyl-4"-yl)-amine (Compound (58))>

9.0 g of biphenyl-4-yl-(4-bromophenyl)-(2'-phenyl-[1,1':4',1"]terphenyl-4"-yl)-amine, 3.9 g of 3-(naphthalene-1-yl)phenylboronic acid, 0.4 g of tetrakis(triphenylphosphine)palladium(0), and 2.6 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from an acetone/methanol mixed solvent to obtain 10.4 g of a white powder of biphenyl-4-yl-{3'-(naphthalene-1-yl)-biphenyl-4-yl}-(2'-phenyl-[1,1':4',1"]terphenyl-4"-yl)-amine (Compound (58), yield: 96.6%).

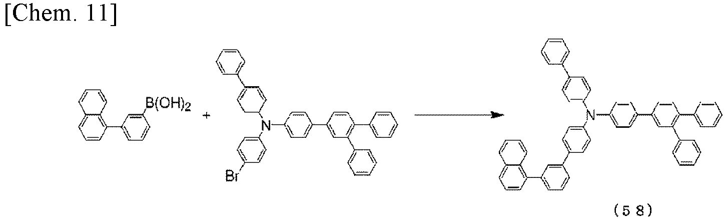

The structure of the obtained white powder was identified using NMR.

The following 41 hydrogen signals were detected using ¹H-NMR (CDCl₃).

δ (ppm) = 7.97 (1H), 7.92 (1H), 7.88 (1H), 7.74 (1H), 7.67 (1H), 7.65 (1H), 7.64 (1H), 7.60 (6H), 7.57-7.49 (6H), 7.49-7.40 (6H), 7.32 (1H), 7.28-7.14 (15H).

### Example 9

### <Synthesis of biphenyl-4-yl-{ 1'-(naphthalene-1-yl)-[1,2' :4',1"]terphenyl-4"-yl}-{4-(naphthalene-2-yl)-phenyl}-amine (Compound (93))>

25.0 g of 1-(5-chloro-biphenyl-2-yl)-naphthalene, 32.4 g of biphenyl-4-yl-{4-(4,4,5,5-tetramethyl-[1,3,2]dioxaborolane-2-yl)-phenyl}-amine, 3.6 g of tris(dibenzylideneacetone)dipalladium(0), 4.5 g of tricyclohexylphosphine, and 50.6 g of tripotassium phosphate were placed in a reaction vessel and stirred in a 1,4-dioxane/H₂O mixed solvent overnight under reflux. After allowing to cool, toluene/H₂O was added to the system, followed by extraction and partition to obtain the organic layer, and the organic layer was concentrated to give a crude product. The crude product was purified through crystallization from a toluene/acetone mixed solvent to obtain 33.8 g of a sand yellow powder of biphenyl-4-yl-{1'-(naphthalene-1-yl)-[1,2':4',1"]terphenyl-4"-yl}-amine (yield: 81.3%).

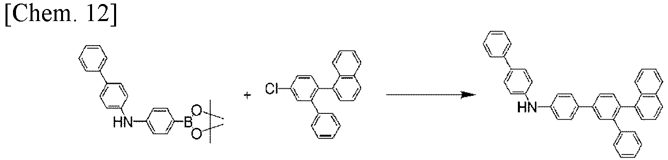

Next, 8.0 g of biphenyl-4-yl-{ 1'-(naphthalene-1-yl)-[1,2':4',1"]terphenyl-4"-yl}-amine, 4.8 g of 2-(4-bromo-phenyl)-naphthalene, 0.1 g of tris(dibenzylideneacetone)dipalladium(0), 0.2 g of tri-tert-butylphosphine, and 1.8 g of sodium tert-butoxide were placed in a reaction vessel and stirred in a toluene solvent for 5 hours under reflux. After allowing to cool, the reaction system was filtered, and the filtrate was concentrated to give a crude product. The crude product was purified through crystallization from a toluene/acetone mixed solvent to obtain 8.8 g of a pale yellow powder of biphenyl-4-yl-{1'-(naphthalene-1-yl)-[1,2':4',1"]terphenyl-4"-yl}-f4-(naphthalene-2-yl)-phenyl}-amine (Compound (93), yield: 79.4%).

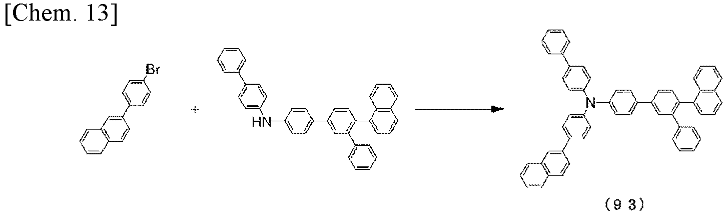

The structure of the obtained pale yellow powder was identified using NMR.

The following 39 hydrogen signals were detected using ¹H-NMR (CDCl₃).

δ (ppm) = 8.08 (1H), 7.94 (2H), 7.90 (1H), 7.85 (1H), 7.80 (3H), 7.77 (1H), 7.71 (5H), 7.65 (2H), 7.59 (2H), 7.53 (2H), 7.47 (4H), 7.42-7.23 (10H), 7.16 (2H), 7.08 (3H).

### Example 10

### <Synthesis of biphenyl-4-yl-{1'-(naphthalene-1-yl)-[1,2':4',1"]terphenyl-4"-yl}-([1,1':4',1"]terphenyl-4"-yl)-amine (Compound (94))>

8.0 g of biphenyl-4-yl-{1'-(naphthalene-1-yl)-[1,2':4',1"]terphenyl-4"-yl}-amine, 5.2 g of 4-bromo-[1,1':4',1"]terphenyl, 0.1 g of tris(dibenzylideneacetone)dipalladium(0), 0.2 g of tri-tert-butylphosphine, and 1.8 g of sodium tert-butoxide were placed in a reaction vessel and stirred in a toluene solvent for 4 hours under reflux. After allowing to cool, the reaction system was filtered, and the filtrate was concentrated to give a crude product. The crude product was purified through recrystallization from an acetone solvent to obtain 10.4 g of a pale yellow powder of biphenyl-4-yl-{1'-(naphthalene-1-yl)-[1,2':4',1"]terphenyl-4"-yl}-([1,1':4',1"]terphenyl-4"-yl)-amine (Compound (94), yield: 90.5%).

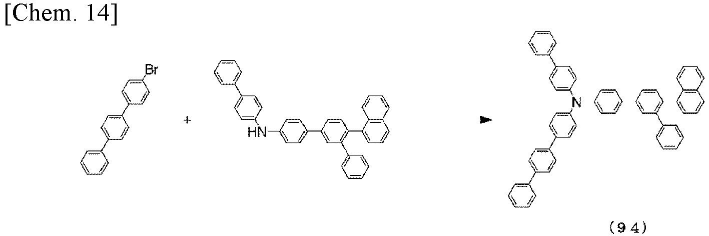

The structure of the obtained pale yellow powder was identified using NMR.

The following 41 hydrogen signals were detected using ¹H-NMR (CDCl₃).

δ (ppm) = 7.82 (1H), 7.78 (1H), 7.75 (2H), 7.71-7.63 (9H), 7.61 (4H), 7.55 (2H), 7.50 (1H), 7.46 (2H), 7.42 (3H), 7.36 (4H), 7.29 (6H), 7.22 (1H), 7.13 (1H), 7.11 (1H), 7.04 (3H).

### Example 11

### <Synthesis of biphenyl-4-yl-{ 1'-(naphthalene-1-yl)-[1,2':4',1"]terphenyl-4"-yl}-{4-(phenanthrene-9-yl)-phenyl}-amine (Compound (96))>

8.0 g of biphenyl-4-yl-{1'-(naphthalene-1-yl)-[1,2':4',1"]terphenyl-4"-yl}-amine, 5.6 g of 9-(4-bromo-phenyl)-phenanthrene, 0.1 g of tris(dibenzylideneacetone)dipalladium(0), 0.2 g of tri-tert-butylphosphine, and 1.8 g of sodium tert-butoxide were placed in a reaction vessel and stirred in a toluene solvent for 4 hours under reflux. After allowing to cool, the reaction system was filtered, and the filtrate was concentrated to give a crude product. The crude product was purified through column chromatography (support: silica gel, eluent: dichloromethane/n-heptane) to obtain 8.1 g of a white powder of biphenyl-4-yl-{1'-(naphthalene-1-yl)-[1,2':4',1"]terphenyl-4"-yl}-{4-(phenanthrene-9-yl)-phenyl}-amine (Compound (96), yield: 68.4%).

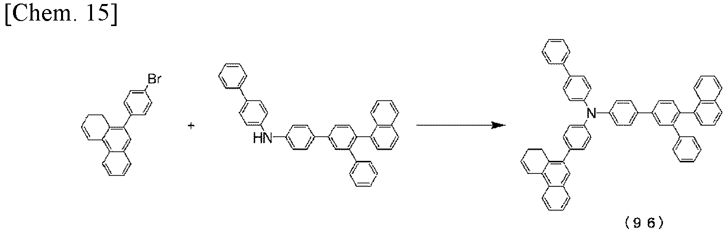

The structure of the obtained white powder was identified using NMR.

The following 41 hydrogen signals were detected using ¹H-NMR (CDCl₃).

δ (ppm) = 8.79 (1H), 8.73 (1H), 8.08 (1H), 7.91 (1H), 7.82 (1H), 7.79 (1H), 7.75 (3H), 7.70 (3H), 7.67 (2H), 7.62 (3H), 7.58 (3H), 7.50 (3H), 7.47-7.30 (12H), 7.22 (1H), 7.13 (2H), 7.04 (3H).

### Example 12

### <Synthesis of {4-(naphthalene-1-yl)-phenyl}-phenyl-(2'-phenyl-[1,1':4',1":4", 1‴]quarterphenyl-4‴-yl)-amine (Compound (148))>

20.0 g of 4'-iodo-[1,1':2',1"]terphenyl, 21.9 g of phenyl-{4'-(4,4,5,5-tetramethyl-[1,3,2]dioxaborolane-2-yl)-biphenyl-4-yl}-amine, 0.7 g of tetrakis(triphenylphosphine)palladium(0), and 11.6 g of potassium carbonate were placed in a reaction vessel and stirred in a toluene/EtOH/H₂O mixed solvent overnight under reflux. After allowing to cool, methanol /H₂O was added to the system, and the mixture was filtered to isolate the solids as a crude product. The crude product was purified through crystallization from a monochlorobenzene/acetone mixed solvent to obtain 23.3 g of a pale red powder of phenyl-(2'-phenyl-[1,1':4',1":4",1‴]quarterphenyl-4‴-yl)-amine (yield: 87.6%).

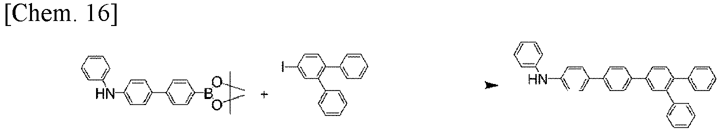

Next, 8.0 g of phenyl-(2'-phenyl-[1,1':4',1":4",1‴]quarterphenyl-4‴-yl)-amine, 5.3 g of 1-(4-bromo-phenyl)-naphthalene, 0.2 g of tris(dibenzylideneacetone)dipalladium(0), 0.2 g of tri-tert-butylphosphine, and 2.0 g of sodium tert-butoxide were placed in a reaction vessel and stirred in a toluene solvent for 4 hours under reflux. After allowing to cool, the reaction system was filtered, and the filtrate was concentrated to give a crude product. The crude product was purified through crystallization from a toluene/acetone mixed solvent to obtain 10.9 g of a pale red powder of {4-(naphthalene-1-yl)-phenyl}-phenyl-(2'-phenyl-[1,1':4',1":4",1‴]quarterphenyl-4‴-yl)-amine (Compound (148), yield: 95.4%).

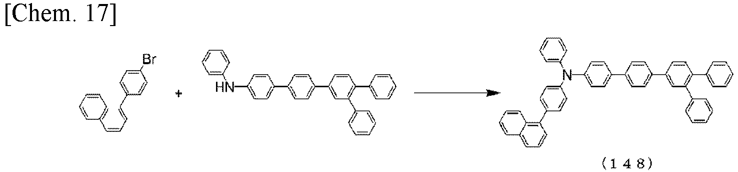

The structure of the obtained pale red powder was identified using NMR.

The following 37 hydrogen signals were detected using ¹H-NMR (CDCl₃).

δ (ppm) = 8.06 (1H), 7.94 (1H), 7.87 (1H), 7.76 (3H), 7.73 (2H), 7.62 (2H), 7.55 (2H), 7.50 (2H), 7.44 (2H), 7.37 (2H), 7.32-7.19 (18H), 7.12 (1H).

### Example 13

### <Synthesis of {4-(naphthalene-2-yl)-phenyl}-phenyl-(2'-phenyl-[1,1':4',1":4",1‴]quarterphenyl-4"' -yl)-amine (Compound (149))>

7.5 g of phenyl-(2'-phenyl-[1,1':4',1":4",1‴]quarterphenyl-4‴-yl)-amine, 4.9 g of 2-(4-bromo-phenyl)-naphthalene, 0.2 g of tris(dibenzylideneacetone)dipalladium(0), 0.2 g of tri-tert-butylphosphine, and 1.8 g of sodium tert-butoxide were placed in a reaction vessel and stirred in a toluene solvent for 4 hours under reflux. After allowing to cool, the reaction system was filtered, and the filtrate was concentrated to give a crude product. The crude product was purified through crystallization from a toluene/acetone mixed solvent to obtain 10.4 g of a white powder of {4-(naphthalene-2-yl)-phenyl}-phenyl-(2'-phenyl-[1,1':4',1":4",1‴]quarterphenyl-4‴-yl)-amine (Compound (149), yield: 97.2%).

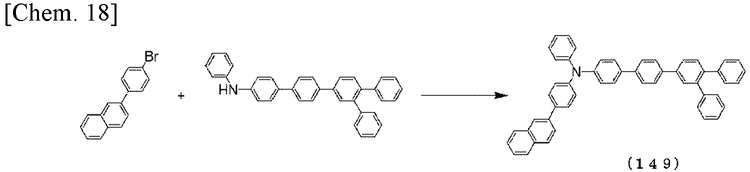

The structure of the obtained white powder was identified using NMR.

The following 37 hydrogen signals were detected using ¹H-NMR (CDCl₃).

δ (ppm) = 8.03 (1H), 7.90 (2H), 7.86 (1H), 7.76 (3H), 7.71 (3H), 7.69 (1H), 7.65 (2H), 7.58 (2H), 7.53 (1H), 7.49 (1H), 7.47 (1H), 7.32 (2H), 7.25-7.16 (16H), 7.09 (1H).

### Example 14

### <Synthesis of {4-(phenanthrene-9-yl)-phenyl}-phenyl-(2'-phenyl-[1,1':4',1":4",1‴]quarterphenyl-4"' -yl)-amine (Compound (150))>

7.5 g of phenyl-(2'-phenyl-[1,1':4',1":4",1‴]quarterphenyl-4‴-yl)-amine, 5.8 g of 9-(4-bromo-phenyl)-phenanthrene, 0.2 g of tris(dibenzylideneacetone)dipalladium(0), 0.2 g of tri-tert-butylphosphine, and 1.8 g of sodium tert-butoxide were placed in a reaction vessel and stirred in a toluene solvent for 5 hours under reflux. After allowing to cool, the reaction system was filtered, and the filtrate was concentrated to give a crude product. The crude product was purified through crystallization from a toluene/acetone mixed solvent to obtain 11.0 g of a white powder of {4-(phenanthrene-9-yl)-phenyl}-phenyl-(2'-phenyl-[1,1':4',1":4",1‴]quarterphenyl-4‴-yl)-amine (Compound (150), yield: 95.7%).

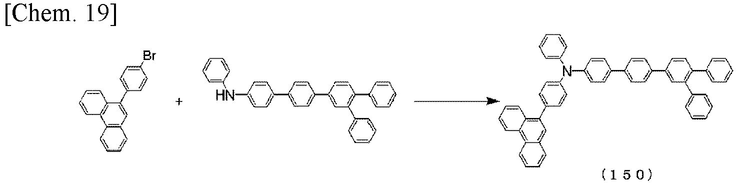

The structure of the obtained white powder was identified using NMR.

The following 39 hydrogen signals were detected using ¹H-NMR (CDCl₃).

δ (ppm) = 8.79 (1H), 8.73 (1H), 8.07 (1H), 7.90 (1H), 7.78-7.56 (13H), 7.53 (1H), 7.47 (2H), 7.25-7.16 (9H), 7.38-7.26 (9H), 7.10 (1H).

### Example 15

<Synthesis of bis{4-(naphthalene-2-yl)-phenyl}-(2'-phenyl-[1,1':4',1"]terphenyl-4"-yl)-amine (Compound (44))>

12.0 g of bis{4-(naphthalene-2-yl)-phenyl}-(3,4-dichloro-biphenyl-4'-yl)-amine, 6.8 g of phenylboronic acid, 0.9 g of tris(dibenzylideneacetone)dipalladium(0), 1.1 g of tricyclohexylphosphine, and 19.8 g of tripotassium phosphate were placed in a reaction vessel and stirred in a 1,4-dioxane/H₂O mixed solvent overnight under reflux. After allowing to cool, the reaction system was filtered, and the filtrate was concentrated to give a crude product. The crude product was purified through column chromatography (support: silica gel, eluent: toluene/n-heptane) to obtain 11.6 g of a white powder of bis{4-(naphthalene-2-yl)-phenyl}-(2'-phenyl-[1,1':4',1"]terphenyl-4"-yl)-amine (Compound (44), yield: 85.5%).

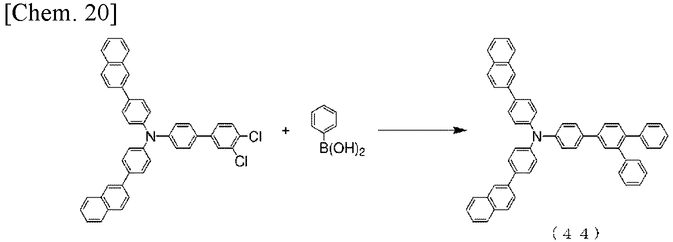

The structure of the obtained white powder was identified using NMR.

The following 39 hydrogen signals were detected using ¹H-NMR (CDCl₃).

δ (ppm) = 8.05 (2H), 7.90 (4H), 7.86 (2H), 7.77 (2H), 7.68 (4H), 7.67 (2H), 7.63 (2H), 7.49 (5H), 7.31 (6H), 7.25-7.14 (10H).

### Example 16

### <Synthesis of biphenyl-4-yl-{1'-(naphthalene-2-yl)-[1,2':4',1"]terphenyl-4"-yl}-phenyl-amine (Compound (103))>

25.0 g of 2-chloro-4-bromo-iodobenzene, 14.2 g of 2-naphthaleneboronic acid, 1.8 g of tetrakis(triphenylphosphine)palladium(0), and 21.8 g of potassium carbonate were placed in a reaction vessel and stirred in a toluene/EtOH/H₂O mixed solvent overnight under reflux. After allowing to cool, toluene/H₂O was added to the system, followed by extraction and partition to obtain the organic layer, and the organic layer was concentrated to give a crude product. The crude product was purified through column chromatography (support: silica gel, eluent: n-heptane) to obtain 18.1 g of a white powder of 3-chloro-4-(naphthalene-2-yl)-bromobenzene (yield: 72.5%).

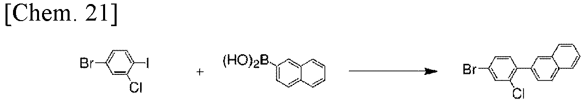

Next, 9.4 g of 3-chloro-4-(naphthalene-2-yl)-bromobenzene, 9.0 g of 4-(biphenyl-4-yl-phenyl-amino)-phenylboronic acid, 0.6 g of tetrakis(triphenylphosphine)palladium(0), and 7.1 g of potassium carbonate were placed in a reaction vessel and stirred in a toluene/EtOH/H₂O mixed solvent overnight under reflux. After allowing to cool, methanol was added to the system, and the mixture was filtered to isolate the solids as a crude product. The crude product was purified through crystallization from an MCB/methanol mixed solvent to obtain 13.4 g of a white powder of biphenyl-4-yl-{3-chloro-4-(naphthalene-2-yl)-biphenyl-4'-yl}-phenyl-amine (yield: 93.0%).

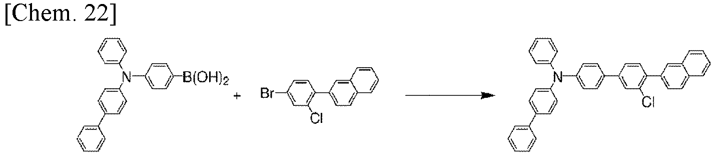

Next, 13.5 g of biphenyl-4-yl-{3-chloro-4-(naphthalene-2-yl)-biphenyl-4'-yl}-phenyl-amine, 3.1 g of phenylboronic acid, 0.7 g of tris(dibenzylideneacetone)dipalladium(0), 0.7 g of tricyclohexylphosphine, and 10.3 g of tripotassium phosphate were placed in a reaction vessel and stirred in a 1,4-dioxane/H₂O mixed solvent overnight under reflux. After allowing to cool, methanol was added to the system, and the mixture was filtered to isolate the solids as a crude product. The crude product was purified through recrystallization from a toluene solvent to obtain 10.0 g of a white powder of biphenyl-4-yl-{1'-(naphthalene-2-yl)-[1,2':4',1"]terphenyl-4"-yl}-phenyl-amine (Compound (103), yield: 68.9%).

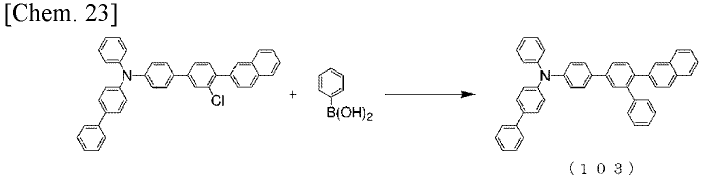

The structure of the obtained white powder was identified using NMR.

The following 33 hydrogen signals were detected using ¹H-NMR (DMSO-d₆).

δ (ppm) = 7.85 (3H), 7.78 (3H), 7.70 (2H), 7.64 (5H), 7.48 (4H), 7.37 (3H), 7.23 (5H), 7.20-7.06 (8H).

The melting point and the glass transition point of each of the compounds synthesized in Examples 2 to 16 were measured using a high-sensitivity differential scanning calorimeter (DSC3100SA manufactured by Bruker AXS K.K.). Also, a vapor-deposited film (thickness: 100 nm) of each of these compound was formed on an ITO substrate, and the work function was measured using an ionization potential measuring system (PYS-202 manufactured by Sumitomo Heavy Industries, Ltd.). The results are collectively shown in Table 1.

**Table 1**

| | | Melting point C° | Glass transition point C° | Work function eV |
|---|---|---|---|---|
| Example 2 | Compound (33) | - | 105 | 5.67 |
| Example 3 | Compound (34) | - | 112 | 5.64 |
| Example 4 | Compound (40) | - | 123 | 5.71 |
| Example 5 | Compound (43) | - | 113 | 5.71 |
| Example 6 | Compound (45) | - | 115 | 5.77 |
| Example 7 | Compound (55) | - | 119 | 5.71 |
| Example 8 | Compound (58) | - | 112 | 5.67 |
| Example 9 | Compound (93) | 243 | 120 | 5.69 |
| Example 10 | Compound (94) | - | 127 | 5.67 |
| Example 11 | Compound (96) | - | 137 | 5.73 |
| Example 12 | Compound (148) | 224 | 106 | 5.76 |
| Example 13 | Compound (149) | - | 104 | 5.72 |
| Example 14 | Compound (150) | - | 122 | 5.82 |
| Example 15 | Compound (44) | - | 108 | 5.65 |
| Example 16 | Compound (103) | 225 | 103 | 5.68 |

The compounds synthesized in Examples 2 to 16 each had a glass transition point of 100°C or more, which means that these compounds are stable in the form of a film.

The compounds synthesized in Examples 2 to 16 had a work function higher than 5.4 eV, which is the work function of common hole transport materials such as NPD and TPD, and thus had a suitable energy level and good hole-transporting capability.

### Example 17

### <Production and Evaluation of Organic EL Device>

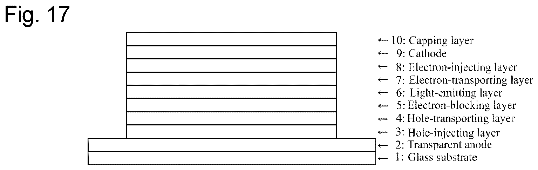

An organic EL device having the configuration as shown in Fig. 17 was prepared in the following manner: a reflective ITO electrode serving as a transparent anode 2 was formed on a glass substrate 1 beforehand, and a hole-injecting layer 3, a hole-transporting layer 4, an electron-blocking layer 5, a light-emitting layer 6, an electron-transporting layer 7, an electron-injecting layer 8, a cathode 9, and a capping layer 10 were vapor-deposited in this order on the ITO electrode.

Specifically, a glass substrate 1 on which an ITO film with a thickness of 50 nm, a reflective silver alloy film with a thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was set inside a vacuum vapor deposition machine, and the pressure was reduced to 0.001 Pa or less.

Subsequently, an electron acceptor (Acceptor-1) having the structural formula below and a compound (HTM-1) having the structural formula below were vapor-deposited so as to coat the transparent anode 2 through binary vapor deposition at vapor deposition rates such that the ratio of the vapor deposition rate of the electron acceptor (Acceptor-1) to the vapor deposition rate of the compound (HTM-1) was 3:97, to thereby form the hole-injecting layer 3 with a thickness of 10 nm.

On the hole-injecting layer 3, the hole-transporting layer 4 (thickness: 140 nm) made of the compound (HTM-1) having the structural formula below was formed.

On the hole-transporting layer 4, the electron-blocking layer 5 (thickness 5 nm) made of Compound (33) obtained in Example 2 was formed.

A compound (EMD-1) having the structural formula below and a compound (EMH-1) having the structural formula below were vapor-deposited on this electron-blocking layer 5 through binary vapor deposition at vapor deposition rates such that the ratio of the vapor deposition rate of the compound (EMD-1) to the vapor deposition rate of the compound (EMH-1) was 5:95, to thereby form the light-emitting layer 6 with a thickness of 20 nm.

A compound (ETM-1) having the structural formula below and a compound (ETM-2) having the structural formula below were vapor-deposited on this light-emitting layer 6 through binary vapor deposition at vapor deposition rates such that the ratio of the vapor deposition rate of the compound (ETM-1) to the vapor deposition rate of the compound (ETM-2) was 50:50, to thereby form the electron-transporting layer 7 with a thickness of 30 nm.

On the electron-transporting layer 7, the electron-injecting layer 8 (thickness: 1 nm) made of lithium fluoride was formed.

On the electron-injecting layer 8, the cathode 9 (thickness: 12 nm) made of a magnesium-silver alloy was formed.

Finally, the capping layer 10 (thickness: 60 nm) made of a compound (CPL-1) having the structure below was formed.

Characteristics of the thus obtained organic EL device were determined in the atmosphere at normal temperature.

Table 2 collectively shows the measurement results of the light emission characteristics of the organic EL device when a DC voltage was applied thereto.

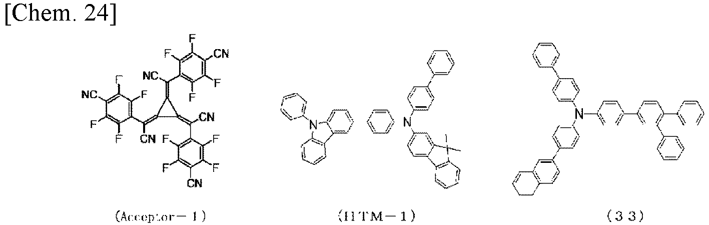

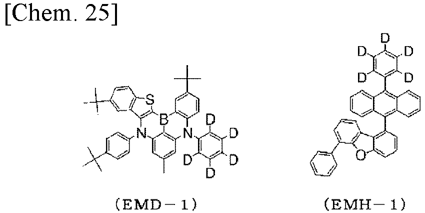

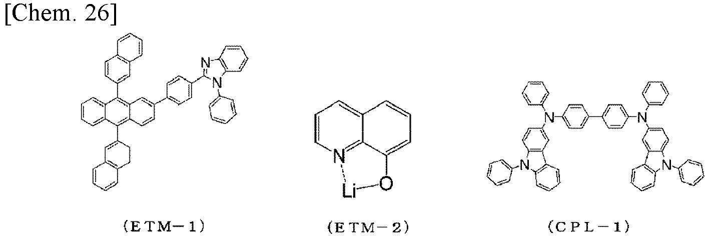

### Example 18

An organic EL device was produced in the same manner as in Example 17, except that Compound (93) obtained in Example 9 was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

### Example 19

An organic EL device was produced in the same manner as in Example 17, except that Compound (148) obtained in Example 12 was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

### Example 20

An organic EL device was produced in the same manner as in Example 17, except that Compound (44) obtained in Example 15 was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

### Example 21

An organic EL device was produced in the same manner as in Example 17, except that Compound (103) obtained in Example 16 was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

### Comparative Example 1

For comparison, an organic EL device was produced in the same manner as in Example 17, except that Compound (HTM-2) having the structural formula below (see Patent Literature 5, for example) was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

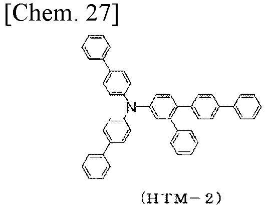

### Comparative Example 2

For comparison, an organic EL device was produced in the same manner as in Example 17, except that Compound (HTM-3) having the structural formula below (see Patent Literature 5, for example) was used as the material for the electron-blocking layer 5 instead of Compound (33) obtained in Example 2. Characteristics of the thus obtained organic EL device were determined in the atmosphere at normal temperature. Table 2 collectively shows the measurement results of the light emission characteristics of the organic EL device when a DC voltage was applied thereto.

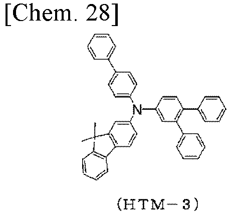

For each of the organic EL devices produced in Examples 17 to 21 and Comparative Examples 1 and 2, the voltage, the luminous efficacy, the power efficiency and the lifespan when a current with a current density of 10 mA/cm² was passed were determined. The measurement results are collectively shown in Table 2. The lifespan was defined as follows: the organic EL device was operated by constant current to emit light at an initial luminance (the luminance when light emission started) of 1000 cd/m², and the time taken for the luminance to decay to 950 cd/m² (corresponding to 95% based on the initial luminance (100%): 95% decay) was determined and used as the lifespan of the device.

**[Table 2]**

| | Electron-blocking layer | Voltage [V] | Luminance [cd/m²] | Luminous efficacy [cd/A] | Power efficiency [lm/W] | Lifespan of device [h] |
|---|---|---|---|---|---|---|
| Example 17 | Compound (33) | 3.32 | 1016 | 10.17 | 9.64 | 492 |
| Example 18 | Compound (93) | 3.35 | 1010 | 10.11 | 9.61 | 425 |
| Example 19 | Compound (148) | 3.34 | 1025 | 10.26 | 9.66 | 512 |
| Example 20 | Compound (44) | 3.35 | 1003 | 10.04 | 9.56 | 438 |
| Example 21 | Compound (103) | 3.33 | 1024 | 10.24 | 9.66 | 467 |
| Com. Ex. 1 | Compound (HTM-2) | 3.42 | 985 | 9.85 | 9.15 | 223 |
| Com. Ex. 2 | Compound (HTM-3) | 3.52 | 934 | 9.34 | 8.61 | 306 |

As is clear from Table 2, the organic EL devices of Comparative Examples 1 and 2 had a luminous efficacy of 9.34 to 9.85 cd/A when a current with a current density of 10 mA/cm² was passed therethrough, and in contrast, the organic EL devices of Examples 17 to 21 had a luminous efficacy of 10.04 to 10.26 cd/A, in other words, higher efficacy. Moreover, while the organic EL devices of Comparative Examples 1 and 2 had a power efficiency of 8.61 to 9.15 lm/W, the organic EL devices of Examples 17 to 21 had a power efficiency as high as 9.56 to 9.66 lm/W. Furthermore, while the organic EL devices of Comparative Examples 1 and 2 had a lifespan (95% decay) of 223 to 306 hours, the organic EL devices of Examples 17 to 21 had a lifespan as long as 425 to 512 hours.

As is clear from the results described above, the organic EL device of the present invention, which includes the arylamine compound having high hole mobility and excellent electron-blocking capability, can have higher luminous efficacy and a longer lifespan than those of conventional organic EL devices.

### Industrial Applicability

The organic EL device of the present invention, which includes an arylamine compound having a specific structure, has increased luminous efficacy and improved durability. Therefore, the organic EL device of the present invention can be applied to a wide variety of uses such as home electric appliances and lighting equipment.

### List of Reference Numerals

- 1: Glass substrate
- 2: Transparent anode
- 3: Hole-injecting layer
- 4: Hole-transporting layer
- 5: Electron-blocking layer
- 6: Light-emitting layer
- 7: Electron-transporting layer
- 8: Electron-injecting layer
- 9: Cathode
- 10: Capping layer

## Claims

1. An arylamine compound represented by a general formula (1) below: 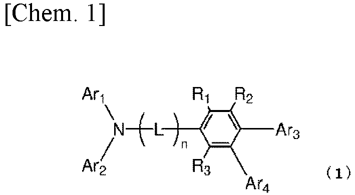
where Ar₁ and Ar₂ are the same or different, and each represent a substituted or unsubstituted aromatic hydrocarbon group, a substituted or unsubstituted aromatic heterocyclic group, or a substituted or unsubstituted fused polycyclic aromatic group;
Ar₃ and Ar₄ are the same or different, and each represent a substituted or unsubstituted phenyl group, a substituted or unsubstituted biphenyl group, a substituted or unsubstituted naphthyl group, a substituted or unsubstituted triphenylsilyl group, a substituted or unsubstituted carbazolyl group, or a substituted or unsubstituted phenanthrenyl group,
L represents a substituted or unsubstituted divalent aromatic hydrocarbon group, a substituted or unsubstituted divalent aromatic heterocyclic group, or a substituted or unsubstituted divalent fused polycyclic aromatic group;
R₁ to R₃ are the same or different, and each represent a hydrogen atom, a deuterium atom, a fluorine atom, a chlorine atom, a cyano group, a nitro group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group; and
n is an integer of 1 or 2, where, when n is 2, L is the same or different.

2. The arylamine compound according to claim 1, wherein R₁ to R₃ in the general formula (1) are the same or different, and each represent a hydrogen atom or a deuterium atom.

3. The arylamine compound according to claim 1 or 2, wherein at least one of Ar₃ and Ar₄ in the general formula (1) represents a substituted or unsubstituted phenyl group, a substituted or unsubstituted biphenyl group, or a substituted or unsubstituted carbazolyl group.

4. The arylamine compound according to any one of claims 1 to 3, wherein Ar₄ in the general formula (1) represents a substituted or unsubstituted phenyl group, a substituted or unsubstituted biphenyl group, or a substituted or unsubstituted carbazolyl group.

5. The arylamine compound according to any one of claims 1 to 4, wherein L in the general formula (1) represents an unsubstituted phenylene group, an unsubstituted biphenylene group, or an unsubstituted naphthylene group.

6. The arylamine compound according to any one of claims 1 to 5, wherein An and Ar₂ in the general formula (1) are the same or different, and each represent a substituted or unsubstituted phenyl group, a substituted or unsubstituted naphthyl group, a substituted or unsubstituted biphenyl group, a substituted or unsubstituted terphenyl group, a substituted or unsubstituted phenanthrenyl group, a substituted or unsubstituted carbazolyl group, a substituted or unsubstituted dibenzofuranyl group, or a substituted or unsubstituted dibenzothienyl group.

7. The arylamine compound according to any one of claims 1 to 6, wherein n in the general formula (1) is 1.

8. An organic electroluminescence device comprising a pair of electrodes and one or more organic layers sandwiched therebetween, wherein at least one of the organic layers contains the arylamine compound according to any one of claims 1 to 7.

9. The organic electroluminescence device according to claim 8, wherein the organic layer is a hole-transporting layer.

10. The organic electroluminescence device according to claim 8, wherein the organic layer is an electron-blocking layer.

11. The organic electroluminescence device according to claim 8, wherein the organic layer is a hole-injecting layer.

12. The organic electroluminescence device according to claim 8, wherein the organic layer is a light-emitting layer.

13. An electronic apparatus comprising a device having a pair of electrodes and one or more organic layers sandwiched therebetween, wherein at least one of the organic layers contains the arylamine compound according to any one of claims 1 to 7.
